# EUROPEAN PATENT APPLICATION

(11) **EP 1 997 889 A2**
(43) Date of publication of application: **03.12.2008**
(21) Application number: 08013208.7
(22) Date of filing: 28.01.2004
(51) Int. Cl.: C12N 15/10, C12Q 1/68

(54) **Method for preparing single-stranded dna libraries**

(30) Priority: 29.01.2003 US 443471 P; 23.04.2003 US 465071 P; 06.06.2003 US 476602 P; 06.06.2003 US 476504 P; 06.06.2003 US 476313 P; 06.06.2003 US 476592 P; 25.08.2003 US 497985 P
(62) Divisional of application: 04706080.1
(71) Applicant: 454 Corporation, Branford, CT 06405 (US)
(72) Inventor: The designation of the inventor has not yet been filed
(74) Representative: Aston, Heidi Frances

(57) **Abstract**

This invention relates to methods of generating single stranded DNA libraries for use in amplification and sequencing reactions. In various aspects, the disclosed methods include: fragmenting DNA; polishing the fragments' ends; lighting the fragments to universal adapters; performing strand displacement and extension of the nicked fragments; purifying the double-stranded libation products; capturing the double-stranded libation products onto a solid support; and isolating single stranded DNA library fragments, and binding these fragments to another solid support.

SEQUENCE LISTING

<110> Costa, Gina L.
Weiner, Michael
Leamon, John
Rothberg, Jonathan

<120> Method For Preparing Single Stranded DNA Libraries

<130> 21465-507 UTIL

<150> US 60/443,471
<151> 2003-01-29

<150> US 60/465,071
<151> 2003-04-23

<150> US 60/476,592
<151> 2003-06-06

<150> US 60/476,504
<151> 2003-06-06

<150> US 60/476,592
<151> 2003-06-06

<150> US 60/476,602
<151> 2003-06-06

<150> US 60/497,985
<151> 2003-08-25

<160> 4

<170> PatentIn version 3.2

<210> 1
<211> 44
<212> DNA
<213> Artificial

<220>
<223> Oligonucleotide

<400> 1
cgtttcccct gtgtgccttg ccatctgttc cctccctgtc atgc 44

<210> 2
<211> 40
<212> DNA
<213> Artificial

<220>
<223> Oligonucleotide

<400> 2
gcatgacagg gagggaacag atggcaaggc acacagggga 40

<210> 3
<211> 40
<212> DNA
<213> Artificial

<220>
<223>  Oligonucleotide

<400>  3
gcatgacacg caacagggga tagggacacg cacgcaacag                                40


<210>  4
<211>  44
<212>  DNA
<213>  Artificial

<220>
<223>  Oligonucleotide

<400>  4
ccatctgttg cgtgcgtgtc cctatcccct gttgcgtgtc atgc                           44

## Description

### RELATED APPLICATIONS

This application claims the benefit of priority to the following applications: USSN 60/443,471 filed January 29, 2003, USSN 60/465,071 filed April 23, 2003; USSN 60/476,504 filed June 6, 2003, USSN 60/476,313 filed June 6, 2003, USSN 60/476,592 filed June 6, 2003, USSN 60/476,602 filed June 6, 2003, USSN 60/476,592 filed June 6, 2003, and USSN 60/497,985 filed August 25, 2003. All patent and patent applications in this paragraph are hereby incorporated herein by reference in their entirety.

This application also incorporates by reference the following copending U.S. patent applications: "Bead Emulsion Nucleic Acid Amplification" filed January 29, 2004, "Bead Emulsion Nucleic Acid Amplification with Continuous Flow" filed January 29, 2004, "Double Ended Sequencing" filed January 29, 2004, and "Methods Of Amplifying And Sequencing Nucleic Acids" filed January 29, 2004.

### FIELD OF THE INVENTION

The invention relates to protein chemistry, molecular biology, and methods of preparing single-stranded libraries for sequence analysis. More specifically, this invention includes methods of processing DNA for use in amplification and sequencing reactions.

### BACKGROUND OF THE INVENTION

In amplification by polymerase chain reaction (PCR), two primers are designed to hybridize to the template DNA at positions complementary to respective primers that are separated on the DNA template molecule by some number of nucleotides. The base sequence of the template DNA between and including the primers is amplified by repetitive complementary strand extension reactions whereby the number of copies of the target DNA fragments is increased by several orders of magnitude. Amplification is exponential as 2", where n equals the number of amplification cycles. Following PCR, the amplified DNA may be sequenced through conventional sequencing methods (see, U.S. Patent No. 6,274,320).

Samples comprising large template DNA or whole DNA genomes comprising long nucleotide sequences are not conducive to efficient amplification by PCR. These long molecules do not naturally possess sequences useful for primer hybridization. In addition, if primer hybridization sequences are added to double stranded DNA molecules, it is difficult to ascertain the directionality of the amplified DNA molecules and this frustrates sequencing efforts.

Various methods have been designed to overcome some of these deficiencies. For example, United States Patent No. 5,508,169 describes that subsets of nucleic acid fragments may be indexed (i.e., selected or targeted) based upon the information contained in non-identical 5'-protruding or 3'-protruding cohesive ends. This includes fragments having 3, 4 or 5 base cohesive ends, such as those revealed by cleavage of DNA by Type II restriction endonucleases and interrupted palindrome recognizing type II restriction endonucleases. The patent describes nucleic acid molecules similar to adaptors (called indexing linkers) which contain protruding single strands complementary to the cohesive ends of cleavage sites of restriction endonucleases (rather than the recognition sequences). Various functional groups or specific nucleic acid sequences designed for particular applications may be selectively attached to the aforementioned subsets of fragments. Selective attachment of indexing linkers having known base sequences in their cohesive ends to a subset of fragments bearing the complementary cohesive ends can be used for the detection, identification, isolation, amplification, and manipulation of the subset of fragments.

United States Patent No. 6,468,748 describes a method of sorting genes and/or gene fragments comprising several steps. First, ds cDNA molecules are prepared from mRNA molecules by reverse transcription, using a poly-T primer optionally having a general primer-template sequence upstream from the poly-T sequence, yielding ds cDNA molecules having the poly-T sequence, optionally having the general primer-template sequence. Second, the ds cDNA molecules are digested with a restriction enzyme that produces digested cDNA molecules with cohesive ends having overhanging ssDNA sequences of a constant number of arbitrary nucleotides. Third, the digested cDNA molecules are ligated to a set of dsDNA oligonucleotide adaptors, each of which adaptors has at one of its ends a cohesive-end ssDNA adaptor sequence complementary to one of the possible overhanging ssDNA sequences of the digested cDNA, at the opposite end a specific primer-template sequence specific for the ssDNA adaptor complementary sequence, and in between the ends a constant sequence that is the same for all of the different adaptors of the set Fourth, the ligated cDNA molecules are amplified by separate polymerase chain reactions, utilizing for each separate polymerase chain reaction a primer that anneals to the cDNA poly-T sequence optionally having the cDNA general primer-template, and a primer from a set of different specific primers that anneal to the cDNA specific primer-template sequences. Fifth, the amplified cDNA molecules are sorted into nonoverlapping groups by collecting the amplification products after each separate polymerase chain reaction, each group of amplified cDNA molecules determined by the specific primer that annealed to the specific primer-template sequence and primed the polymerase chain reaction.

United States Patent No. 5,863,722 describes a method and materials for sorting polynucleotides with oligonucleotide tags. The oligonucleotide tags are capable of hybridizing to complementary oligomeric compounds consisting of subunits having enhanced binding strength and specificity as compared to natural oligonucleotides. Such complementary oligomeric compounds are referred to as "tag complements." Subunits of tag complements may consist of monomers of non-natural nucleotide analogs, referred to as "antisense monomers" or they may comprise oligomers having lengths in the range of 3 to 6 nucleotides or analogs thereof, including antisense monomers, the oligomers being selected from a minimally cross-hybridizing set. In such a set, a duplex made up of an oligomer of the set and the complement of any other oligomer of the set contains at least two mismatches. In other words, an oligomer of a minimally cross-hybridizing set at best forms a duplex having at least two mismatches with the complement of any other oligomer of the same set. Tag complements attached to a solid phase support are used to sort polynucleotides from a mixture of polynucleotides each containing a tag. The surface of each support is derivatized by only one type of tag complement which has a particular sequence. Similarly, the polynucleotides to be sorted each comprise an oligonucleotide tag in the repertoire, such that identical polynucleotides have the same tag and different polynucleotides have different tags. Thus, when the populations of supports and polynucleotides are mixed under conditions which permit specific hybridization of the oligonucleotide tags with their respective complements, subpopulations of identical polynucleotides are sorted onto particular beads or regions. The subpopulations of polynucleotides can then be manipulated on the solid phase support by micro-biochemical techniques.

United States Patent No. 5,728,524 describes a process for the categorization of nucleic acid sequences in which these sequences are linked to a population of adaptor molecules, each exhibiting specificity for linking to a sequence including a predetermined nucleotide base. The resulting linked sequences are then categorized based upon selection for the particular base.

However, the art does not describe methods for generating libraries of unknown fragment sequences additionally comprising two known sequences, each different than the other, one being adjoined at each end. Thus, a need exists for a method which overcomes shortcomings of the prior art. Accordingly, the present invention is directed to describing such methods, materials, and kits as required to facilitate manipulation of multiple DNA sequences in a sample.

### BRIEF SUMMARY OF THE INVENTION

This invention describes a novel method for preparing a library of multiple nucleic acid sequences from a sample where the library is suited to further quantitative and comparative analysis, particularly where the multiple nucleic acid sequences are unknown and derived from large template DNA or whole (or partial) genome DNA. In certain embodiments of the invention, sequences of single stranded DNA (ssDNA) are prepared from a sample of large template DNA or whole or partial DNA genomes through fragmentation, polishing, adaptor ligation, nick repair, and isolation of ssDNA.

Therefore, in one aspect, the present invention provides a method for clonally isolating a library comprising a plurality of ssDNAs, wherein each ssDNA comprises a first single stranded universal adaptor and a second single stranded universal adaptor, the method comprising:
(a) fragmenting large template DNA molecules to generate a plurality of fragmented DNA molecules;
(b) attaching a first or second universal double stranded adaptor to a first end of each fragmented DNA molecule and a first or second universal adaptor to a second end of each fragmented DNA molecule to form a mixture of adaptor ligated DNA molecules;
(c) isolating a plurality of single stranded DNA molecules each comprising a first single stranded universal adaptor and a second single stranded universal adaptor; and
(d) delivering the single stranded DNA molecules into reactors such that a plurality of the reactors include one DNA molecule, thereby clonally isolating the library.

In certain aspects, the single stranded DNA molecules are delivered into droplets in a water-in-oil emulsion (i.e., microreactors), or onto multiwell surfaces (e.g., PicoTiter plates). The single stranded DNA molecules may be delivered via attachment to a solid support (e.g., beads).

In other aspects, the adaptor ligated DNA molecules comprising a first double stranded universal adaptor and second double stranded universal adaptor is attached to a solid support via one strand of the double stranded universal adaptor (via the first or second universal adaptor). The adaptor ligated DNA molecules which have not attached to a solid support are washed away, and one strand of the adaptor ligated DNA molecules is released. This generates a mixture comprising a plurality of ssDNAs comprising a population of single stranded molecules with a first and second universal adaptor pair, thereby generating a library.

The sequence of the fragmented DNA may be known or unknown. In a preferred embodiment, the sequence of the fragmented DNA, particularly the sequence of the ends of the fragmented DNA, is unknown.

In another aspect, the present invention includes a method for generating a ssDNA library linked to solid supports comprising: (a) generating a library of ssDNA templates; (b) attaching the ssDNA templates to solid supports; and (c) isolating the solid supports on which one ssDNA template is attached. In still another aspect, the present invention includes a library of mobile solid supports made by the method disclosed herein.

### BRIEF DESCRIPTION OF THE FIGURES

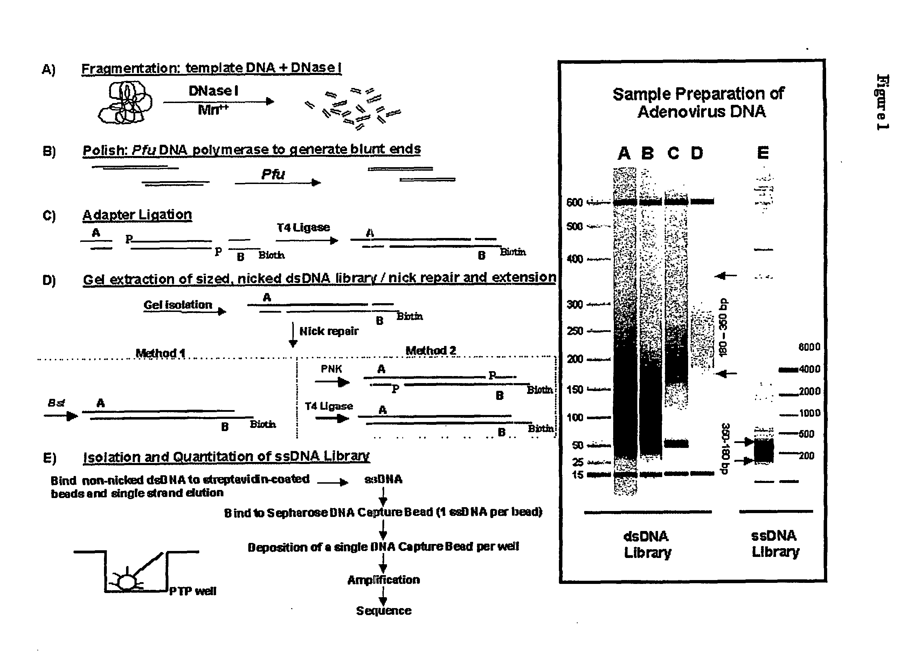

Figure 1 is a schematic representation of the entire process of library preparation including the steps of template DNA fragmentation (Figure 1A), end polishing (Figure 1B), adaptor ligation (Figure 1C), nick repair, strand extension and gel isolation (Figure 1D). Figure 1 also depicts a representative agarose gel containing a sample preparation of a 180-350 base pair adenovirus DNA library according to the methods of this invention.
Figure 2A is a schematic representation of the universal adaptor design according one embodiment of the present invention. Each universal adaptor is generated from two complementary ssDNA oligonucleotides that are designed to contain a 20 bp nucleotide sequence for PCR priming, a 20 bp nucleotide sequence for sequence priming and a unique 4 bp discriminating sequence comprised of a non-repeating nucleotide sequence (*i.e.,* ACGT, CAGT, etc.).
Figure 2B depicts a representative universal adaptor sequence pair for use with the invention. Adaptor A sense strand: SEQ ID NO:1; Adaptor A antisense strand: SEQ ID NO:2; Adaptor B sense strand: SEQ ID NO:3; Adaptor B antisense strand: SEQ ID NO:4.
Figure 2C is a schematic representation of universal adaptor design for use with the invention.

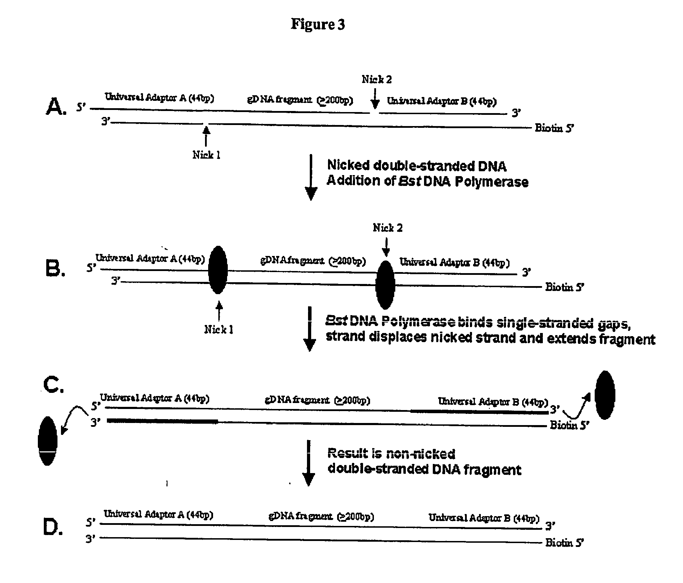

Figure 3 represents the strand displacement and extension of nicked double-stranded DNA fragments according to the present invention. Following the ligation of universal adaptors generated from synthetic oligonucleotides, double-stranded DNA fragments will be generated that contain two nicked regions following T4 DNA ligase treatment (Figure 3A). The addition of a strand displacing enzyme (i.e., *Bst* DNA polymerase I) will bind nicks (Figure 3B), strand displace the nicked strand and complete nucleotide extension of the strand (Figure 3C) to produce non-nicked double-stranded DNA fragments (Figure 3D).

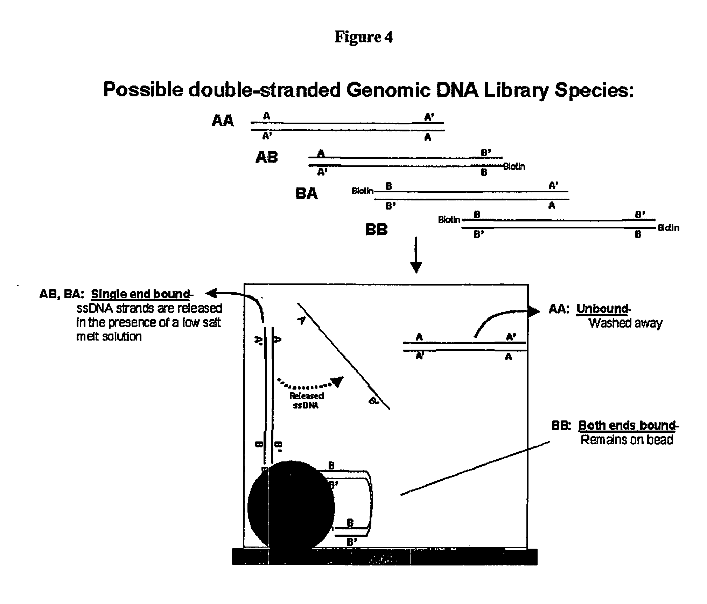

Figure 4 represents the isolation of directionally-ligated single-stranded DNA according to the present invention using streptavidin-coated magnetic beads. Following ligation with universal adaptors A and B (the two different adaptors are sometimes referred to as a "first" and "second" universal adaptor), double-stranded DNA will contain adaptors in four possible combinations: AA, BB, AB, and BA. When universal adaptor B contains a 5'-biotin, magnetic streptavidin-coated solid supports are used to capture and isolate the AB, BA, and BB populations (population AA is washed away). The BB population is retained on the beads as each end of the double-stranded DNA is attached to a bead and is not released. However, upon washing in the presence of a low salt buffer, only populations AB and BA will release a single-stranded DNA fragment that is complementary to the bound strand. Single-stranded DNA fragments are isolated from the supernatant and used as template for subsequent applications. This method is described below in more detail.
Figure 5A represents an insert flanked by PCR primers and sequencing primers.
Figure 6 represents truncated, product produced by PCR primer mismatch at cross-hybridization region (CHR).
Figures 7A-7D depict the assembly for the nebulizer used for the methods of the invention. A tube cap was placed over the top of the nebulizer (Figure 7A) and the cap was secured with a nebulizer clamp assembly (Figure 7B). The bottom of the nebulizer was attached to the nitrogen supply (Figure 7C) and the entire device was wrapped in parafilm (Figure 7D).
Figure 8 depicts representative BioAnalyzer output from analysis of a single stranded DNA library.
Figure 9A depicts representative results for LabChip analysis of a single stranded DNA library following nebulization and polishing.
Figure 9B depicts representative size distribution results for an adaptor-ligated single stranded DNA library following nebulization, polishing, and gel purification.
Figure 10 depicts the calculation for primer candidates based on melting temperature.

### DETAILED DESCRIPTION OF THE INVENTION

This invention relates to the preparation of sample DNA for amplification and sequencing reactions. The invention includes a method for preparing the sample DNA comprised of the following steps: (a) fragmenting large template DNA or whole genomic DNA samples to generate a plurality of digested DNA fragments; (b) creating compatible ends on the plurality of digested DNA samples; (c) ligating a set of universal adaptor sequences onto the ends of fragmented DNA molecules to make a plurality of adaptor-ligated DNA molecules, wherein each universal adaptor sequence comprises a PCR primer sequence, a sequencing primer sequence and a discriminating key sequence and wherein one adaptor is attached to biotin; (d) separating and isolating the plurality of ligated DNA fragments; (e) removing any portion of the plurality of ligated DNA fragments; (f) nick repair and strand extension of the plurality of ligated DNA fragments; (g) attaching each of the ligated DNA fragments to a solid support; and (h) isolating populations comprising single-stranded adaptor-ligated DNA fragments for which there is a unique adaptor at each end (i.e., providing directionality).

Unless otherwise defined, all technical and scientific terms used herein have the same meaning as commonly understood by one of ordinary skill in the art to which this invention belongs. Methods and materials similar or equivalent to those described herein can be used in the practice of the present invention, and exemplified suitable methods and materials are described below. For example, methods may be described which comprise more than two steps. In such methods, not all steps may be required to achieve a defined goal and the invention envisions the use of isolated steps to achieve these discrete goals. The disclosures of all publications, patent applications, patents, and other references are incorporated *in toto* herein by reference. In addition, the materials, methods, and examples are illustrative only and not intended to be limiting.

As used herein, the term "universal adaptor" refers to two complementary and annealed oligonucleotides that are designed to contain a nucleotide sequence for PCR priming and a nucleotide sequence for sequence priming. Optionally, the universal adaptor may further include a unique discriminating key sequence comprised of a non-repeating nucleotide sequence (i.e., ACGT, CAGT, etc.). A set of universal adaptors comprises two unique and distinct double-stranded sequences that can be ligated to the ends of double-stranded DNA. Therefore, the same universal adaptor or different universal adaptors can be ligated to either end of the DNA molecule. When comprised in a larger DNA molecule that is single stranded or when present as an oligonucleotide, the universal adaptor may be referred to as a single stranded universal adaptor.

As used herein, the term "discriminating key sequence" refers to a sequence including a combination of the four deoxyribonucleotides (i.e., A, C, G, T). The same discriminating sequence can be used for an entire library of DNA fragments. Alternatively, different discriminating key sequences can be used to track libraries of DNA fragments derived from different organisms. Longer discriminating key sequences can be used for a mixture of more than one library.

As used herein, the term "plurality of molecules" refers to DNA isolated from the same source, whereby different organisms may be prepared separately by the same method. In one embodiment, the plurality of DNA samples is derived from large segments of DNA, e.g., genomic DNA, cDNA, viral DNA, plasmid DNA, cosmid DNA, artificial chromosome DNA (e.g., BACs, YACs, MACs, PACs), synthetic DNA, phagemid DNA, phasemid DNA, or from reverse transcripts of viral RNA. This DNA may be derived from any source, including any mammal (i.e., human, nonhuman primate, rodent, or canine), plant, bird, reptile, fish, fungus, bacteria, or virus.

As used herein, the term "library" refers to a subset of smaller sized DNA species generated from a larger DNA template, e.g., a segmented or whole genome.

As used herein, the term "unique", as in "unique PCR priming regions" refers to a sequence that does not exist or exists at an extremely low copy level within the DNA molecules to be amplified or sequenced.

As used herein, the term "compatible" refers to an end of double stranded DNA to which an adaptor molecule may be attached (i.e., blunt end or cohesive end).

As used herein, the term "fragmenting" refers to a process by which a larger molecule of DNA is converted into smaller pieces of DNA.

As used herein, "large template DNA" would be DNA of more than 5 kb, 10 kb, or 25 kb, preferably more than 500 kb, more preferably more than 1 MB, and most preferably 5 MB or larger.

As used herein, the term "stringent hybridization conditions" refers to those conditions under which only fully complimentary sequences will hybridize to each other.

The following discussion summarizes the basic steps involved in the methods of the invention. The steps are recited in a specific order, however, as would be known by one of skill in the art, the order of the steps may be manipulated to achieve the same result Such manipulations are contemplated by the inventors. Further, some steps may be minimized as would also be known by one of skill in the art

### Fragmentation

In the practice of the methods of the present invention, the fragmentation of the DNA sample can be done by any means known to those of ordinary skill in the art. Preferably, the fragmenting is performed by enzymatic, chemical, or mechanical means. The mechanical means may include sonication, French Press, HPLC, HydroShear DNA shearing device (GeneMachines, San Carlos, CA), and nebulisation. The enzymatic means may be performed by digestion with Deoxyribonuclease I (DNase I), non-specific nucleases, or single or multiple restriction endonucleases. In a preferred embodiment, the fragmentation results in ends for which the sequence adjacent to the end is not known. The sequence adjacent to the end may be at least 5 bases, 10 bases, 20 bases, 30 bases, or 50 bases.

### Enzymatic Fragmentation

In a preferred embodiment, the enzymatic means is DNase I. DNase I is a versatile enzyme that nonspecifically cleaves double-stranded DNA (dsDNA) to release 5'-phosphorylated oligonucleotide products. DNase I has optimal activity in buffers containing Mn²⁺, Mg²⁺ and Ca²⁺. The purpose of the DNase I digestion step is to fragment a large DNA genome into smaller species comprising a library. The cleavage characteristics of DNase I will result in random digestion of template DNA (*i.e.*, minimal sequence bias) and in the predominance of blunt-ended dsDNA fragments when used in the presence of manganese-based buffers (Melgar, E. and D.A. Goldthwait 1968. Deoxyribonucleic acid nucleases. II. The effects of metal on the mechanism of action of Deoxyribonuclease L J. Biol. Chem. 243: 4409). The range of digestion products generated following DNase I treatment of genomic templates is dependent on three factors: i) amount of enzyme used (units); ii) temperature of digestion (°C); and iii) incubation time (minutes). The DNase I digestion conditions outlined below have been optimized to yield genomic libraries with a size range from 50-700 base pairs (bp).

In a preferred embodiment, DNase I is used to digest large template DNA or whole genome DNA for 1-2 minutes to generate a population of oligonucleotides that range from 50 to 500 bp, or 50 to 700 bp. In another preferred embodiment, the DNase I digestion is performed at a temperature of 10°C-37°C. In yet another preferred embodiment, the digested DNA fragments are 50 bp to 700 bp in length.

### Mechanical Fragmentation

Another preferred method for nucleic acid fragmentation is mechanical fragmentation. Mechanical fragmentation methods include sonication and nebulization, and use of HydroShear, HPLC, and French Press devices. Sonication may be performed by a tube containing DNA in a suitable buffer (i.e., 10 mM Tris, 0.1 mM EDTA) and sonicating for a varying number of 10 second bursts using maximum output and continuous power Sonicators are commercially available from, e.g., Misonix Inc. (Farmingdale, NY), and can be used essentially as described by Bankier and Barrell (Bankier, A.T., Weston, K.M., and Barrell, B.G., "Random cloning and sequencing by the M13/dideoxynucleotide chain termination method", Meth. Enzymol. 155, 51-93 (1987). For sonication, it is preferred to maintain the nucleic acid at a uniform temperature by keeping the sample on ice. Constant temperature conditions, at 0° C for example, are preferred to maintain an even fragment distribution. The optimal conditions for sonication may be determined empirically for a given DNA sample before preparative sonication is performed. For example, aliquots of DNA can be treated for different times under sonication and the size and quality of DNA can be analyzed by PAGE. Once optimal sonication conditions are determined, the remaining DNA can be sonicated according to those pre-determined conditions.

Another preferred method for nucleic acid fragmentation is treatment by nebulizers (e.g., protocols, and hardware available from GeneMachines, San Carlos, California. Also see U.S. Patents 5,506,100 and 5,610,010). In nebulization, hydrodynamic shearing forces are used to fragment DNA strands. For example, DNA in a aqueous solution can be passed through a tube with an abrupt contraction. As the solution approaches the contraction, the fluid accelerates to maintain the volumetric flow rate through the smaller area of the contraction. During this acceleration, drag forces stretch the DNA until it snaps. Optionally, the DNA solution can be passed several times (e.g., 15 to 20 cycles) through the contraction until the fragments are too short for further shearing. By adjusting the contraction and the flow rate of the fluid, the size of the final DNA fragment may be determined. Software for controlling and monitoring reaction conditions is available to allow automation of the nebulizing process. As another advantage, there are no special buffer requirements for nebulization . For example, DNA may be suspended in various solutions including, but not limited to, water, Tris buffer, Tris-EDTA buffer, and Tris-EDTA with up to 0.5 M NaCl.

### Polishing

Polishing digestion of genomic DNA (gDNA) templates with DNase I in the presence of Mn²⁺ produces fragments of DNA that are either blunt-ended or have protruding termini with one or two nucleotides in length. Similarly, fragmentation of DNA by mechanical means provides a combination of fragments with blunt-ends or overhanging ends. These DNA fragments, whether generated enzymatically or mechanically, may be "polished" using the procedure described below.

Polishing (also called end repair) refers to the conversion of non-blunt ended DNA into blunt ended DNA. In one method, polishing may be performed by treatment with a single strand-specific exonuclease, such as BAL32 nuclease or Mung Bean nuclease. Generally, the nuclease should be calibrated prior to use.

In one embodiment, blunt ends are created with *Pfu* DNA polymerase. In other embodiments, blunt ends are created with other DNA polymerases such as T4 DNA polymerase or Klenow DNA polymerase. *Pfu* "polishing" or blunt ending can be used to increase the amount of blunt-ended species generated following genomic template digestion with DNase I. *Pfu* DNA polymerase fills in 5' overhangs. Additionally, *Pfu* DNA polymerase exhibits 3'→ 5' exonuclease activity. Thus, the enzyme can be used to remove single and double nucleotide extensions to further increase the amount of blunt-ended DNA fragments available for adaptor ligation (see, e.g., Costa, G.L. and M.P. Weiner. 1994a. Protocols for cloning and analysis of blunt-ended PCR-generated DNA fragments. PCR Methods Appl 3(5):S95; Costa, G.L., A. Grafsky and M.P. Weiner. 1994b. Cloning and analysis of PCR-generated DNA fragments. PCR Methods Appl 3(6):338; Costa, G.L. and M.P. Weiner. 1994c. Polishing with T4 or Pfu polymerase increases the efficiency of cloning of PCR products. Nucleic Acids Res. 22(12):2423).

### Adaptor Ligation

Following fragmentation and blunt ending of the DNA library, universal adaptor sequences can be added to each DNA fragment In various embodiments of the invention, the universal adaptors are designed to include: 1) a set of unique PCR priming regions that are typically 10-20 bp in length (any suitable size may be used) located adjacent to; 2) a set of unique sequencing priming regions that are typically 10-20 bp in length (any suitable size may be used) optionally followed by; 3) a unique discriminating key sequence (e.g., 1-12 bp in length) including a combination of at least one of each of the four deoxyribonucleotides (i.e., A, C, G, T). In a preferred embodiment, the discriminating key sequence is 4 bases in length. In another embodiment, the discriminating key sequence may be combinations of 1-4 bases. In another embodiment, the key sequence includes one of each of the four nucleotides. In certain embodiments, the key sequence includes one or more ribonucleotides, e.g., U.

In one embodiment of the invention, each unique universal adaptor is forty-four bp (44 bp) in length, although any suitable size may be used. In a preferred embodiment the universal adaptors are ligated, using T4 DNA ligase, onto each end of the DNA fragment to generate a total nucleotide addition of 88 bp to each DNA fragment, although any suitable size may be used. Different universal adaptors can be specifically designed for each DNA library preparation and therefore provide a unique identifier for each organism. For example, different library preparations can employ different key sequences. It is understood that the size and sequence of the universal adaptors may be modified as would be apparent to one of skill in the art. Thus, the adaptors for use with the invention are not limited to the size and sequence described herein.

For example, to prepare two distinct universal adaptors (i.e., "first" and "second"), single-stranded oligonucleotides may be ordered from a commercial vendor (e.g., Integrated DNA Technologies, IA or Operon Technologies, CA). In certain embodiments of the invention, all of the first adaptors for a library share one nucleotide sequence, including a PCR priming sequence, sequencing primer sequence, and discriminating key sequence, while all of the second adaptors share another nucleotide sequence. In another embodiment, the universal adaptor oligonucleotide sequences are modified during synthesis with one or more phosphorothioate linkages in place of phosphodiester linkages. For example, adaptor oligonucleotides can include two or three phosphorothioate linkages at both the 5' and 3' ends, or at one end. Unmodified oligonucleotides are typically subject to rapid degradation by contaminating nucleases that catalyze the hydrolytic cleavage of the phosphodiester linkage between nucleotide bases. One simple and widely used nuclease-resistant chemistry available for use in oligonucleotide applications is the phosphorothioate modification. In phosphorothioates, a sulfur atom replaces a non-bridging oxygen in the oligonucleotide backbone making it resistant to all forms of nuclease digestion (i.e. resistant to both endonuclease and exonuclease digestion). Each oligonucleotide is HPLC-purified to ensure there are no contaminating or spurious oligonucleotide sequences in the synthetic oligonucleotide preparation.

The universal adaptors are designed to allow directional ligation to the fragmented DNA. Each set of double-stranded universal adaptors is designed with a PCR priming region that includes non-complementary 5' four-base overhangs which are unable to ligate to each other or to the blunt-ended DNA fragment. Thus, binding occurs between the 3' end of the adaptor and the 5' end of the DNA fragment or between the 3' end of the DNA fragment and the 5' end of the adaptor. Double-stranded universal adaptor sequences are generated using single-stranded oligonucleotides that are designed with sequences that allow primarily complimentary oligonucleotides to anneal, and to prevent cross-hybridization between two non-complimentary oligonucleotides.

In one embodiment, 95% of the universal adaptors are formed from the annealing of complimentary oligonucleotides. In a preferred embodiment, 97% of the universal adaptors are formed from the annealing of complimentary oligonucleotides. In a more preferred embodiment, 99% of the universal adaptors are formed from the annealing of complimentary oligonucleotides. In a most preferred embodiment, 100% of the universal adaptors are formed from the annealing of complimentary oligonucleotides. Exemplary methods of primer design to minimize cross-hybridization between the primers and spurious target sequences are provided in Example 2.

In certain aspects of the invention, an overhanging nucleotide (e.g., T) is added to the blunt 3' ends of the first and second adaptors. In parallel, a polymerase is used to add an overhanging nucleotide (e.g., A) to the blunt 5' ends of the template DNA. The overhanging nucleotides of the adaptors and template are complementary to allow more efficient adaptor ligation.

In other aspects, a plasmid capture system is used in accordance with the disclosed methods. For example, the double stranded universal adaptors can be inserted into a plasmid. The adaptor region can include the following sequences, in order: Restriction Site(s), PCR Primer Sequence 1, Sequencing Primer Sequence 1, Key Sequence 1, Restriction Site(s), Key Sequence 2, Sequencing Primer Sequence 2, PCR Priming Sequence 2, and Restriction Site(s). In one approach, the plasmid is digested with one or more restriction enzymes that cut between Key Sequence 1 and Key Sequence 2. The fragmented template DNA is ligated between Key Sequence 1 and Key Sequence 2. The ligated construct is then digested to cut after PCR Priming Sequence 2. The digested end next to PCR Priming Sequence 2 is filled in with biotinylated nucleotides. The biotinylated construct is digested to cut prior to PCR Primer Sequence 1. The Adaptor-DNA fragment-Adaptor-biotin segment is excised and isolated, e.g., by binding to a streptavidin magnetic bead. Other embodiments of the plasmid capture system are also possible through application of known cloning techniques. These embodiments are also encompassed by the invention.

One of the two adaptors can be linked to a support binding moiety. In a preferred embodiment, a.5' biotin is added to the first universal adaptor to allow subsequent isolation of ssDNA template and noncovalent coupling of the universal adaptor to the surface of a solid support that is saturated with a biotin-binding protein (e.g., streptavidin, NeutrAvidinTM or avidin). Suitable supports include but are not limited to magnetic beads, affinity columns, membranes (e.g., PDVF membrane, nitrocellulose, etc.), which can be coated with streptavidin or another member of a binding pair. Other linkages are well known in the art and may be used in place of biotin-streptavidin. For example, anti-body/antigen-epitope, receptor/ligand and oligonucleotide pairing or complimentarity linkages can be used. In one embodiment, the solid support is a bead, preferably a polystyrene bead. In one preferred embodiment, the bead has a diameter of about 2.8 µm, although any suitable size may be used. In another preferred embodiment, the bead is a paramagnetic bead (e.g., Dynal Biotech, Inc., Lake Success, NY). As used herein, this bead is referred to as a "sample prep bead".

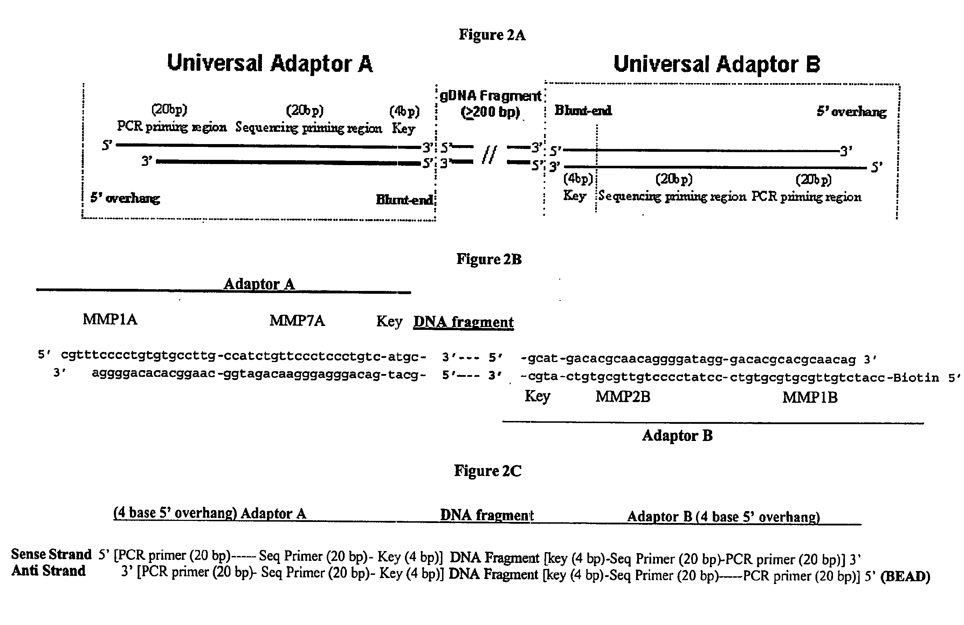

Each universal adaptor may be prepared by combining and annealing two ssDNA oligonucleotides, one containing the sense sequence and the second containing the antisense (complementary) sequence. Schematic representation of the universal adaptor design is depicted in Figure 2.

### Isolation of Ligation Products

The universal adaptor ligation results in the formation of fragmented DNAs with adaptors on each end, unbound single adaptors, and adaptor dimers. In a preferred embodiment, agarose gel electrophoresis is used as a method to separate and isolate the adapted DNA library population from the unligated single adaptors and adaptor dimer populations. In other embodiments, the fragments may be separated by size exclusion chromatography, filtration, sucrose sedimentation, or other nucleic acid separation techniques known to those skilled in the art. The procedure of DNase I digestion of DNA typically yields a library population that ranges from 50-700 bp. In a preferred embodiment, upon conducting agarose gel electrophoresis in the presence of a DNA marker, the addition of the 88 bp universal adaptor set will shift the DNA library population to a larger size and will result in a migration profile in the size range of approximately 130-800 bp; adaptor dimers will migrate at 88 bp; and adaptors not ligated will migrate at 44 bp. Therefore, numerous double-stranded DNA libraries in sizes ranging from 200-800 bp can be physically isolated from the agarose gel and purified using standard gel extraction techniques. In one embodiment, gel isolation of the adapted ligated DNA library will result in the recovery of a library population ranging in size from 200-500 bp. Other methods of distinguishing adaptor-ligated fragments are known to one of skill in the art.

### Nick Repair

Because the DNA oligonucleotides used for the universal adaptors are not 5' phosphorylated, gaps will be present at the 3' junctions of the fragmented DNAs following ligase treatment (see Figure 3A). These "gaps" or "nicks" can be filled in by using a DNA polymerase enzyme that can bind to, strand displace, and extend the nicked DNA fragments. DNA polymerases that lack 3'→ 5' exonuclease activity but exhibit 5' → 3' exonuclease activity have the ability to recognize nicks, displace the nicked strands, and extend the strand in a manner that results in the repair of the nicks and in the formation of non-nicked double-stranded DNA (see Figure 3B and 3C) (Hamilton, S.C., J.W. Farchaus and M.C. Davis. 2001. DNA polymerases as engines for biotechnology. BioTechniques 31:370*).*

Several modifying enzymes are utilized for the nick repair step, including but not limited to polymerases, ligases, and kinases. DNA polymerases that can be used in the methods of the invention include, for example, *E. coli* DNA polymerase I, *Thermoanaerobacter thermohydrosulfuricus* polymerase I, and bacteriophage phi 29. In a preferred embodiment, the strand displacing enzyme *Bacillus stearothermophilus* polymerase I (*Bst* DNA polymerase I) is used to repair the nicked dsDNA and results in non-nicked dsDNA (see Figure 3D). In another preferred embodiment, the ligase is T4 DNA ligase and the kinase is T4 polynucleotide kinase.

### Isolation of Single-Stranded DNA

Following the generation of non-nicked dsDNA, ssDNAs comprising both the first and second adaptor molecules may be isolated. Double-stranded DNA libraries will have adaptors bound in the following configurations:
Universal Adaptor A - DNA fragment - Universal Adaptor A
Universal Adaptor B - DNA fragment - Universal Adaptor A*
Universal Adaptor A - DNA fragment - Universal Adaptor B*
Universal Adaptor B - DNA fragment - Universal Adaptor B

"A" and "B" correspond to the first and second adaptors. The desired populations are designated with asterisks.

Preferably, the universal adaptors are designed such that only one universal adaptor has a 5' biotin moiety. For example, if universal adaptor B has a 5'biotin moiety, streptavidin-coated sample prep beads can be used to bind all double-stranded DNA library species with universal adaptor B. Genomic library populations that contain two universal adaptor A species will not contain a 5' biotin moiety and will not bind to streptavidin-containing sample prep beads and thus can be washed away. The only species that will remain attached to beads are those with universal adaptors A and B and those with two universal adaptor B sequences.

DNA species with two universal adaptor B sequences (i.e., biotin moieties at each 5'end) will be bound to streptavidin-coated sample prep beads at each end, as each strand comprised in the double strand will be bound. Double-stranded DNA species with a universal adaptor A and a universal adaptor B will contain a single 5'biotin moiety and thus will be bound to streptavidin-coated beads at only one end. Where the sample prep beads are magnetic, the beads will remain coupled to a solid support when magnetized. Accordingly, in the presence of a low-salt ("melt" or denaturing) solution, only those DNA fragments that contain a single universal adaptor A and a single universal adaptor B sequence will release the complementary unbound strand. This single-stranded DNA population attached to beads may be collected and quantitated by, for example, pyrophosphate sequencing, real-time quantitative PCR, agarose gel electrophoresis, fluorescent dye binding assay (PicoGreen®; Molecular Probes, Inc., Eugene, OR), or capillary gel electrophoresis.

In one embodiment, ssDNA libraries that are created according to the methods of the invention are quantitated to calculate the number of molecules per unit volume. For example, the molecules can be annealed to a solid support that includes oligonucleotide capture primers that are complementary to the PCR priming regions of the universal adaptor ends of the ssDNA species.

In certain embodiments, beads comprising capture primers annealed to ssDNA library molecules can be transferred to a thermocycler to allow PCR amplification. Clonal populations of single species of single stranded DNA captured on DNA beads may then sequenced. In one embodiment, the solid support is a bead, preferably a sepharose bead. As used herein, this bead is referred to as a "DNA capture bead".

The beads used herein may be of any convenient size and fabricated from any number of known materials. Example of such materials include: inorganics, natural polymers, and synthetic polymers. Specific examples of these materials include: cellulose, cellulose derivatives, acrylic resins, glass; silica gels, polystyrene, gelatin, polyvinyl pyrrolidone, copolymers of vinyl and acrylamide, polystyrene cross-linked with divinylbenzene or the like (see, Merrifield Biochemistry 1964; 3, 1385-1390), polyacrylamides, latex gels, polystyrene, dextran, rubber, silicon, plastics, nitrocellulose, celluloses, natural sponges, silica gels, glass, metals plastic, cellulose, cross-linked dextrans (e.g., Sephadex^{™}) and agarose gel (Sepharose^{™}) and solid phase supports known to those of skill in the art. In one embodiment, the diameter of the DNA capture bead is 20-70 µm. In a preferred embodiment, the diameter of the DNA capture bead is 20-50 µm. In a more preferred embodiment, the diameter of the DNA capture bead is about 30 µm.

In one aspect, the invention includes a method for generating a library of solid supports comprising: (a) preparing a population of ssDNA templates according to the methods disclosed herein; (b) attaching each DNA template to a solid support such that there is one molecule of DNA per solid support; (c) amplifying the population of single-stranded templates such that the amplification generates a clonal population of each DNA fragment on each solid support; (d) sequencing clonal populations of the ssDNA templates.

In one embodiment, the solid support is a DNA capture bead. In another embodiment, the DNA is genomic DNA, cDNA, or reverse transcripts of RNA (e.g., viral RNA). The DNA may be attached to the solid support, for example, via a biotin-streptavidin linkage, a covalent linkage, or by complementary oligonucleotide hybridization. In one embodiment, each DNA template is ligated to a set of universal adaptors. In another embodiment, the universal adaptor pair comprises a PCR primer sequence, a sequencing primer sequence, and a discriminating key sequence. Single-stranded DNAs with unique ends are isolated and then attached to a solid support and exposed to amplification techniques for clonal amplification. The DNA may be amplified by PCR. In one aspect, the invention provides a library attached to solid supports made by the methods described herein.

The DNA prepared by this method may be used for many molecular biological procedures, such as linear extension, rolling circle amplification, PCR, and sequencing. The linkage reaction can be driven, for example, by using a high molar ratio of bead to DNA. The capture of single-stranded DNA molecules follows a Poisson distribution and results in subsets of beads having no DNA attached, one molecule of DNA attached, or more than one molecule of DNA attached. In a preferred embodiment, there is one molecule of DNA attached to each bead. In addition, it is possible to include additional modifications with the adaptors that may be useful for further manipulations of the isolated library.

### Binding Nucleic Acid Template to Capture Beads

In certain embodiments of the invention, a single stranded nucleic acid template to be amplified is attached to a capture bead. The nucleic acid template may be attached to the solid support capture bead in any manner known in the art. Numerous methods exist in the art for attaching DNA to a solid support such as the preferred microscopic bead. According to the present invention, covalent chemical attachment of the DNA to the bead can be accomplished by using standard coupling agents, such as water-soluble carbodiimide, to link the 5'-phosphate on the DNA to amine-coated capture beads through a phosphoamidate bond. Another alternative is to first couple specific oligonucleotide linkers to the bead using similar chemistry, and to then use DNA ligase to link the DNA to the linker on the bead. Other linkage chemistries to join the oligonucleotide to the beads include the use of N-hydroxysuccinamide (NHS) and its derivatives. In such a method, one end of the oligonucleotide may contain a reactive group (such as an amide group) which forms a covalent bond with the solid support, while the other end of the linker contains a second reactive group that can bond with the oligonucleotide to be immobilized. In a preferred embodiment, the oligonucleotide is bound to the DNA capture bead by covalent linkage. However, non-covalent linkages, such as chelation or antigen-antibody complexes, may also be used to join the oligonucleotide to the bead.

Oligonucleotide linkers can be employed which specifically hybridize to unique sequences at the end of the DNA fragment, such as the overlapping end from a restriction enzyme site or the "sticky ends" of bacteriophage lambda based cloning vectors, but blunt-end ligations can also be used beneficially. These methods are described in detail in US 5,674,743. It is preferred that any method used to immobilize the beads will continue to bind the immobilized oligonucleotide throughout the steps in the methods of the invention.

In one embodiment, each capture bead is designed to have a plurality of nucleic acid primers that recognize (i.e., are complementary to) a portion of the nucleic template, and the nucleic acid template is thus hybridized to the capture bead. In the methods described herein, clonal amplification of the template species is desired, so it is preferred that only one unique nucleic acid template is attached to any one capture bead.

The beads used herein may be of any convenient size and fabricated from any number of known materials. Example of such materials include: inorganics, natural polymers, and synthetic polymers. Specific examples of these materials include: cellulose, cellulose derivatives, acrylic resins, glass, silica gels, polystyrene, gelatin, polyvinyl pyrrolidone, copolymers of vinyl and acrylamide, polystyrene cross-linked with divinylbenzene or the like (as described, e.g., in Merrifield, Biochemistry 1964, 3, 1385-1390), polyacrylamides, latex gels, polystyrene, dextran, rubber, silicon, plastics, nitrocellulose, natural sponges, silica gels, control pore glass, metals, cross-linked dextrans (e.g., Sephadex^{™}) agarose gel (Sepharose^{™}), and solid phase supports known to those of skill in the art. In a preferred embodiment, the capture beads are Sepharose beads approximately 25 to 40 µm in diameter.

### Emulsification

For use with the present invention, capture beads with or without attached nucleic acid template may be suspended in a heat stable water-in-oil emulsion. It is contemplated that a plurality of the microreactors include only one template and one bead. There may be many droplets that do not contain a template or which do not contain a bead. Likewise there may be droplets that contain more than one copy of a template. The emulsion may be formed according to any suitable method known in the art. One method of creating emulsion is described below but any method for making an emulsion may be used. These methods are known in the art and include adjuvant methods, counter-flow methods, cross-current methods, rotating drum methods, and membrane methods. Furthermore, the size of the microcapsules may be adjusted by varying the flow rate and speed of the components. For example, in dropwise addition, the size of the drops and the total time of delivery may be varied. Preferably, the emulsion contains a density of about 3,000 beads encapsulated per microliter.

Various emulsions that are suitable for biologic reactions are referred to in Griffiths and Tawfik, EMBO, 22, pp. 24-35 (2003); Ghadessy et al., Proc. Natl. Acad. Sci. USA 98, pp. 4552-4557 (2001); United States Patent No. 6,489,103 and WO 02/22869, each fully incorporated herein by reference. It is noted that Griffiths et al., (U.S. Pat. No. 6,489,103 and WO 99/02671) refers to a method for *in vitro* sorting of one or more genetic elements encoding a gene products having a desired activity. This method involves compartmentalizing a gene, expressing the gene, and sorting the compartmentalized gene based on the expressed product. In contrast to the present invention, the microencapsulated sorting method of Griffith is not suitable for parallel analysis of multiple microcapsules because their nucleic acid product is not anchored and cannot be anchored. Since the nucleic acids of Griffiths are not anchored, they would be mixed together during demulsification.

The emulsion is preferably generated by adding beads to an amplification solution. As used herein, the term "amplification solution" means the sufficient mixture of reagents that is necessary to perform amplification of template DNA. One example of an amplification solution, a PCR amplification solution, is provided in the Examples below. It will be appreciated that various modifications may be made to the amplification solution based on the type of amplification being performed and whether the template DNA is attached to the beads or provided in solution. In one embodiment, the mixture of beads and amplification solution is added dropwise into a spinning mixture of biocompatible oil (e.g., light mineral oil, Sigma) and allowed to emulsify. In another embodiment, the beads and amplification solution are added dropwise into a cross-flow of biocompatible oil. The oil used may be supplemented with one or more biocompatible emulsion stabilizers. These emulsion stabilizers may include Atlox 4912, Span 80, and other recognized and commercially available suitable stabilizers. In preferred aspects, the emulsion is heat stable to allow thermal cycling, e.g., to at least 94°C, at least 95°C, or at least 96°C. Preferably, the droplets formed range in size from about 5 microns to about 500 microns, more preferably from about 10 microns to about 350 microns, even more preferably from about 50 to 250 microns, and most preferably from about 100 microns to about 200 microns. Advantageously, cross-flow fluid mixing allows for control of the droplet formation, and uniformity of droplet size. We note that smaller water droplets not containing beads may be present in the emulsion.

The microreactors should be sufficiently large to encompass sufficient amplification reagents for the degree of amplification required. However, the microreactors should be sufficiently small so that a population of microreactors, each containing a member of a DNA library, can be amplified by conventional laboratory equipment, e.g., PCR thermocycling equipment, test tubes, incubators and the like. Notably, the use of microreactors allows amplification of complex mixtures of templates (e.g., genomic DNA samples or whole cell RNA) without intermixing of sequences, or domination by one or more templates (e.g., PCR selection bias; see, Wagner et al., 1994, Suzuki and Giovannoni, 1996; Chandler et al., 1997, Polz and Cavanaugh, 1998).

With the limitations described above, the optimal size of a microreactor may be on average 100 to 200 microns in diameter. Microreactors of this size would allow amplification of a DNA library comprising about 600,000 members in a suspension of microreactors of less than 10 ml in volume. For example, if PCR is the chosen amplification method, 10 ml of microreactors would fit into 96 tubes of a regular thermocycler with 96 tube capacity. In a preferred embodiment, the suspension of 600,000 microreactors would have a volume of less than 1 ml. A suspension of less than 1 ml may be amplified in about 10 tubes of a conventional PCR thermocycler. In a most preferred embodiment, the suspension of 600,000 microreactors would have a volume of less than 0.5 ml.

Another embodiment of the invention is directed to a method of performing nucleic acid amplification with a template and a bead, but without attachment of the template to the bead. In one aspect, the bead may comprise a linker molecule that can bind the amplified nucleic acid after amplification. For example, the linker may be a linker that can be activated. Such linkers are well known and include temperature sensitive or salt sensitive binding pairs such as streptavidin/biotin and antibodies/antigen. The template nucleic acid may be encapsulated with a bead and amplified. Following amplification, the amplified nucleic acid may be linked to the beads, e.g., by adjustments in temperature or salt concentration.

### Amplification

The template nucleic acid may be amplified, while attached or unattached to beads, by any suitable method of amplification including transcription-based amplification systems (Kwoh D. et al., Proc. Natl. Acad Sci. (U.S.A.) 86:1173 (1989); Gingeras T. R. et al., WO 88/10315; Davey, C. et al., EP Publication No. 329,822; Miller, H. I. et al., WO 89/06700), "RACE" (Frohman, M. A., In: PCR Protocols: A Guide to Methods and Applications, Academic Press, NY (1990)) and one-sided PCR (Ohara, O. et al., Proc. Natl. Acad. Sci. (U.S.A.) 86.5673-5677 (1989)). Still other methods such as di-oligonucleotide amplification, isothermal amplification (Walker, G. T. et al., Proc. Natl. Acad. Sci. (U.S.A.) 89:392-396 (1992)), Nucleic Acid Sequence Based Amplification (NASBA; see, e.g., Deiman B et al., 2002, Mol Biotechnol. 20(2):163-79), whole-genome amplification (see, e.g., Hawkins TL et al., 2002, Curr Opin Biotechnol. 13(1):65-7), strand-displacement amplification (see, e.g., Andras SC, 2001, Mol Biotechnol. 19(1):29-44), rolling circle amplification (reviewed in U.S. Pat. No. 5,714,320), and other well known techniques may be used in accordance with the present invention. In certain aspects, a nucleic acid template is amplified after encapsulation with a bead in a microreactor. Alternatively, a nucleic acid template is amplified after distribution onto a multiwell surface, e.g., a PicoTiter plate.

In a preferred embodiment, DNA amplification is performed by PCR. PCR according to the present invention may be performed by encapsulating the target nucleic acid with a PCR solution comprising all the necessary reagents for PCR. Then, PCR may be accomplished by exposing the emulsion to any suitable thermocycling regimen known in the art. In a preferred embodiment, 30 to 50 cycles, preferably about 40 cycles, of amplification are performed. It is desirable, but not necessary, that following the amplification procedure there be one or more hybridization and extension cycles following the cycles of amplification. In a preferred embodiment, 10 to 30 cycles, preferably about 25 cycles, of hybridization and extension are performed (e.g., as described in the examples). Routinely, the template DNA is amplified until typically at least 10,000 to 50,000,000 copies are immobilized on each bead. It is recognized that for nucleic acid detection applications, fewer copies of template are required. For nucleic acid sequencing applications we prefer that at least two million to fifty million copies, preferably about ten million to thirty million copies of the template DNA are immobilized on each bead. The skilled artisan will recognize that the size of bead (and capture site thereon) determines how many captive primers can be bound (and thus how many amplified templates may be captured onto each bead).

In one aspect, the invention encompasses a method for clonally isolating a library comprising a plurality of single stranded DNA molecules comprising: a) fragmenting large template DNA molecules to generate a plurality of fragmented DNA molecules; b) attaching a first or second universal double stranded adaptor to a first end of each fragmented DNA molecule and a first or second universal adaptor to a second end of each fragmented DNA molecule to form a mixture of adaptor ligated DNA molecules; c) isolating a plurality of single stranded DNA molecules each comprising a first single stranded universal adaptor and a second single stranded universal adaptor to obtain a library; and d) delivering the single stranded DNA molecules into reactors such that a plurality of the reactors include one DNA molecule, thereby clonally isolating the library.

In another aspect, the invention encompasses a method for generating a library comprising a plurality of single stranded DNA molecules, comprising: a) fragmenting large or whole genomic template DNA molecules to generate a plurality of fragmented DNA molecules; b) ligating a first universal double stranded adaptor or a second universal adaptor to a first end of each fragmented DNA molecule and a first universal adaptor or second universal adaptor to a second end of each fragmented DNA molecule to produce a mixture of adaptor ligated DNA molecules, wherein the first universal adaptor contains a moiety that binds to a solid support; c) attaching to a solid support those DNA molecules comprising a first double stranded universal adaptor, d) washing away adaptor ligated DNA molecules which have not attached to the solid support; e) strand separating those adaptor ligated DNA molecules that are attached to the solid support to release a plurality of single stranded DNA molecules comprising a first single stranded universal adaptor at one end and a second single stranded adaptor at the other end; and f) isolating the single stranded DNA molecules, thereby generating a library. In specific aspects, (f) can be accomplished by: i) delivering the single stranded DNA molecules onto a location on a reactor array; or ii) delivering the single stranded DNA molecules into droplets in a water-in-oil emulsion.

According to these methods, the first universal double stranded adaptor or the second universal adaptor can be attached to the fragmented DNA molecules by ligation. For example, DNA ligase may be used. These methods can further include a step of repairing single stranded nicks in the mixture of adaptor ligated DNA molecules using DNA repair and modifying enzymes, such as a polymerase, ligase, kinase, or combinations thereof. As particular examples, the enzymes can include *Bacillus stearothermophilus* polymerase I, T4 ligase, and T4 polynucleotide kinase. The template DNA for these methods can comprise genomic DNA, cDNA, plasmid DNA, cosmid DNA, artificial chromosome DNA, synthetic DNA, phasemid DNA, phagemid DNA, or reverse transcripts. Fragmenting may be performed by enzymatic, chemical, or mechanical means. For example, DNase I enzyme can be used in a digestion performed at a temperature of 10-37°C for 1-2 minutes. Alternatively, a restriction enzyme may be used. The mechanical means can be a nebulizer, French Press, sonicator, or a HydroShear.

For use with these methods, the fragmented DNA molecules can be 50 bp to 700 bp in length. The compatible ends can be blunt ends, or the compatible ends can include an A or T overhang. Blunt ends can be created with an enzyme such as *Pfu* polymerase, T4 DNA polymerase, and Klenow fragments. The first or second double stranded universal adaptor may comprise one or more phosphorothioate linkages, and may be attached to a biotin moiety. In addition, the first double stranded universal adaptors or the second double stranded universal adaptors or both double stranded universal adaptors may comprise a discriminating key sequence. For example, the discriminating key sequence is 3-12 nucleotides in length and comprise at least one nucleotide selected from the group consisting of A, G, C, U, and T. The first and second double stranded universal adaptor may comprise a PCR priming sequence and a sequencing primer sequence. In various aspects, the PCR priming sequence is 10-20 base pairs in length, and the sequencing primer sequence is 10-20 base pairs in length. In addition, the PCR priming sequence and the sequencing primer sequence may overlap.

In accordance with these methods, the mixture of adaptor ligated DNA molecules is separated by a method selected from the group consisting of gel electrophoresis, filtration, size exclusion chromatography, and sucrose sedimentation. The plurality of single stranded DNA molecules may be obtained by a treatment selected from the group consisting of low salt treatment, high pH treatment, and chemical denaturation treatment. In further aspects, the plurality of single stranded DNA molecules may be attached to a DNA capture bead. In addition, the DNA capture bead may comprise a component of a binding pair, such as avidin/biotin, ligand/receptor, antigen/antibody or complementary nucleotides. Preferably, the DNA capture bead is a paramagnetic bead.

The invention also encompasses a method for generating a single stranded DNA library attached to solid supports comprising: a) generating a plurality of single stranded DNA templates; b) attaching each of the plurality of ssDNA templates to a solid support; and c) isolating the solid supports on which the single stranded DNA templates are attached.

The invention further encompasses a method for generating a single stranded DNA library attached to solid supports comprising: a) fragmenting large template DNA molecules to generate a plurality of fragmented DNA molecules; b) attaching a first or second universal double stranded adaptor to a first end of each fragmented DNA molecule and a first or second universal adaptor to a second end of each fragmented DNA molecule to make a mixture of adaptor ligated DNA molecules; c) isolating those single stranded DNA molecules which comprise a first single stranded universal adaptor and a second single stranded universal adaptor; and d) attaching the isolated single stranded molecules from (c) to a solid support.

For use with this method, the solid support may be a DNA capture bead, and the DNA may be genomic DNA, cDNA, plasmid DNA, cosmid DNA, artificial chromosome DNA, synthetic DNA, phasemid DNA, or phagemid DNA. In certain aspects the DNA is attached to the solid support via a binding pair such as avidin/biotin, ligand/receptor, antigen/antibody and complementary nucleotides. Also encompassed is a library of mobile solid supports made by this method.

Additionally, the invention comprises a nucleic acid molecule comprising a first adaptor, a fragment of template DNA, and a second adaptor, wherein the first adaptor and second adaptor each comprise a sequencing primer, a PCR primer, and a discriminating key sequence, and wherein the first adaptor and second adaptor, when dissociated, do not cross-hybridize to each other under stringent hybridization conditions. In this method, the PCR primer may be 10-20 base pairs in length, the sequencing primer may be 10-20 base pairs in length, and the discriminating key sequence may be 3 to 12 base pairs in length. As examples, the template DNA can be genomic DNA, cDNA plasmid DNA, cosmid DNA, artificial chromosome DNA, synthetic DNA, phasemid DNA, or phagemid DNA. Preferably, the nucleic acid molecule, when dissociated, has minimal cross-hybridization to dissociated template DNA.

Also encompassed by the invention is a method for preparing single stranded DNA molecules, comprising: a) fragmenting large or whole genomic template DNA molecules to generate a plurality of fragmented DNA molecules; b) ligating a first universal double stranded adaptor or a second universal adaptor to a first end of each fragmented DNA molecule and a first universal adaptor or second universal adaptor to a second end of each fragmented DNA molecule to produce a mixture of adaptor ligated DNA molecules; c) attaching adaptor ligated DNA molecules comprising a first double stranded universal adaptor and a second double stranded adaptor to a solid support via one strand of the first double stranded universal adaptor; d) washing away adaptor ligated DNA molecules which have not attached to the solid support; e) strand separating those adaptor ligated DNA molecules that are attached to the solid support to release a plurality of single stranded DNA molecules comprising a first single stranded universal adaptor at one end and a second single stranded adaptor at the other end; and; and f) isolating the single stranded DNA molecules.

Further encompassed by the invention is a method for delivering nucleic acid templates to a plurality of reaction centers comprising the steps of: a) providing a population of nucleic acid templates; b) isolating each nucleic acid template from the population to a sequestering agent to form a population of sequestered nucleic acid templates; and c) delivering the population of sequestered nucleic acid templates to the plurality of reaction centers wherein each reaction center receives one sequestered nucleic acid. For this method, the isolating step may comprise attaching the nucleic acid templates to a bead, or encapsulating the nucleic acid template in an emulsion of a water-in-oil emulsion. The nucleic acid template may be encapsulated with a bead that can bind the nucleic acid. In this method, the delivering step may comprise delivering the sequestered nucleic acid to a plurality of reaction centers, wherein each reaction center is a well on a picotiter plate. The method can further comprising the step of attaching the isolated single stranded molecules each individually to a solid support.

Other features of the invention will become apparent in the course of the following description of exemplary embodiments which are given for illustration of the invention and are not intended to be limiting thereof. Throughout this specification, various patents, published patent applications and scientific references are cited to describe the state and content of the art.

### EXAMPLES

### Example 1: Sample Preparation

### DNA Sample:

The DNA should be of high quality and free from contaminants such as proteins, nucleases, lipids, and other chemicals (such as residual EDTA from preparation) and salts. It is preferred that genomic DNA should have a 260/280 ratio of 1.8 or higher. If it is desired to sequence the genome of only one organism, then the DNA should be quality checked to ensure that there is no contaminating DNA. For example: a preparation of human DNA may be checked by PCR to ensure that it is not contaminated by bacterial DNA molecules. Another method of checking for contamination is by restriction digestion patterns and especially restriction digestion followed by Southern Blot using suitable probes known to be specific for an organism (e.g., human or mouse) and a second probe known to be specific for a possible contaminating organism (e.g., *E. coli).* If it is desired, the DNA should originate from a single clone of the organism (e.g., a colony if from bacteria).

### Step 1: DNase I Digestion

The purpose of the DNase I digestion step is to fragment a large stretch of DNA such as a whole genome or a large portion of a genome into smaller species. This population of smaller-sized DNA species generated from a single DNA template is referred to as a "library". Deoxyribonuclease I (DNase I) is an endonuclease which cleaves double-stranded template DNA. The cleavage characteristics of DNase I allow random digestion of template DNA (i.e., minimal sequence bias) and will result in the predominance of blunt-ended, double-stranded DNA fragments when used in the presence of manganese-based buffers (Melgar and Goldthwait 1968). The digestion of genomic templates by DNase I is dependent on three factors: i) quantity of enzyme used (units); ii) temperature of digestion (°C); and iii) incubation time (minutes). The DNase I digestion conditions outlined below were optimized to yield DNA libraries in a size range from 50-700 base pairs (bp).
1. DNA was obtained and prepared to a concentration of 0.3 mg/ml in Tris-HCl (10mM, pH 7-8). A total of 134 µl of DNA (15 µg) was needed for this preparation. It is recommended to not use DNA preparations diluted with buffers containing EDTA (i.e., TE, Tris/EDTA). The presence of EDTA is inhibitory to enzyme digestion with DNase I. If the DNA preparation contains EDTA, it is important that the DNA be "salted" out of solution and reconstituted with the appropriate Tris-HCl buffer (10 mM, pH 7-8) or nanopure H₂O (pH 7-8).
2. In a 0.2 ml tube, DNase I Buffer, comprising 50 µl Tris pH 7.5 (1M), 10 µl MnCl₂ (1M), 1 µl BSA (100 mg/ml), and 39 µl water was prepared.
3. In a separate 0.2 ml tube, 15 µl of DNase I Buffer and 1.5 µl of DNase I (1U/ml) was added. The reaction tube was placed in a thermal cycler set to 15°C.
4. The 134 µl of DNA (0.3 mg/ml) was added to the DNase I reaction tube placed in the thermal cycler set at 15°C. The lid was closed and the sample was incubated for exactly 1 minute. Following incubation, 50 µl of 50 mM EDTA was added to stop the enzyme digestion.
5. The digested DNA was purified by using the QiaQuick PCR purification kit. The digestion reaction was then split into four aliquots, and four spin columns were used to purify each aliquot (37.5 µl per spin column). Each column was eluted with 30 µl elution buffer (EB) according to the manufacturer's protocol. The eluates were then combined to generate a final reaction volume of 120 µl.
6. One 3 µl aliquot of the digestion reaction was saved for analysis using a BioAnalzyer DNA 1000 LabChip.

### Step 2: Pfu Polishing

Digestion of DNA templates with DNase I yields fragments of DNA that are primarily blunt-ended, however, some fragments will have ends that contain protruding termini that are one or two nucleotides in length. *Pfu* polishing is used to increase the amount of blunt-ended species by fill-in (i.e., "blunting") of 5' overhangs. Additionally, *Pfu* DNA polymerase has 3'→ 5' exonuclease activity that will result in the removal of single and double nucleotide extensions. *Pfu* polishing increases the amount of blunt-ended DNA fragments available for adaptor ligation (Costa 1994a, 1994b, 1994c). The following *Pfu* polishing protocol was used.
1. In a 0.2 ml tube, 115 µl purified, DNase I-digested DNA fragments, 15 µl 10X Cloned *Pfu* buffer, 5 µl dNTPs (10 mM), and 15 µl cloned *Pfu* DNA polymerase (2.5 U/µl) were added in order.
2. The polishing reaction components were mixed well and incubated at 72° C for 30 minutes.
3. Following incubation, the reaction tube was removed and placed on ice for 2 minutes.
4. The polishing reaction mixture was then split into four aliquots and purified using QiaQuick PCR purification columns (37.5 µL on each column). Each column was eluted with 30 µl buffer EB according to the manufacturer's protocol. The eluates were then combined to generate a final reaction volume of 120 µL.
5. One 3 µl aliquot of the final polishing reaction was saved for analysis using a BioAnalzyer DNA 1000 LabChip.

### Step 3: Ligation of Universal Adaptors to Fragmented DNA Library

Following fragmentation and polishing of the genomic DNA library, primer sequences are added to the ends of each DNA fragment These primer sequences are termed "Universal Adaptors" and are comprised of double-stranded oligonucleotides that contain specific priming regions that afford both PCR amplification and nucleotide sequencing. The Universal Adaptors are designed to include a set of unique PCR priming regions that are 20 base pairs in length located adjacent to a set of unique sequencing priming regions that are 20 base pairs in length, followed by a unique 4-base "key" consisting of one of each deoxyribonucleotide (i.e., A, C, G, T). Each unique Universal Adaptor (termed "Universal Adaptor A" and "Universal Adaptor B") is forty-four base pairs (44 bp) in length. Universal Adaptors are ligated, using T4 DNA ligase, onto each end of the DNA fragment to generate a total nucleotide addition of 88 bp to each DNA fragment. Different Universal Adaptors are designed specifically for each genomic DNA library preparation and will therefore provide a unique identifier for each organism.

To prepare a pair of Universal Adaptors, single-stranded oligonucleotides are designed in-house and are manufactured through a commercial vendor. Universal Adaptor DNA oligonucleotides are designed with two phosphorothioate linkages at each oligonucleotide end that serve to protect against nuclease activity (Samini, T.D., B. Jolles, and A. Laigle. 2001. Best minimally modified antisense oligonucleotides according to cell nuclease activity. Antisense Nucleic Acid Drug Dev. 11(3):129., the disclosure of which is incorporated *in toto* herein by reference.). Each oligonucleotide is HPLC-purified to ensure there are no contaminating or spurious DNA oligonucleotide sequences in the final prep.

The Universal Adaptors are designed to allow directional ligation to the blunt-ended, fragmented genomic DNA. For each Universal Adaptor pair, the PCR priming region contains a 5' four-base overhang and a blunt-ended 3' Key region. Directionality is achieved as the blunt-end side of the Universal Adaptor ligates to the blunt-ended DNA fragment while the 5' overhang of the adaptor cannot ligate to the blunt-ended DNA fragment. Additionally, a 5' biotin is added to the Universal Adaptor B to allow subsequent isolation of ssDNA template (Step 8). Each Universal Adaptor is prepared by annealing, in a single tube, the two single-stranded complementary DNA oligonucleotides (i.e., one oligo containing the sense sequence and the second oligo containing the antisense sequence). The following ligation protocol was used.
1. In a 0.2 ml tube, 39 µl nH₂O (molecular biology grade water), 25 µl digested, polished DNA Library, 100 µl 2X Quick Ligase Reaction Buffer, 20 µl MMP1 (10 pm/µl) adaptor set, 100:1 ratio, and 16 µl Quick Ligase were added in order. The ligation reaction was mixed well and incubated at RT for 20 minutes.
2. The ligation reaction was then removed and a 10-µl aliquot of the ligation reaction was purified for use on the BioAnalyzer. A single spin column from the Qiagen Min-Elute kit was used. The column was eluted with 10 µl EB according to the procedure per manufacturers' protocol. A 1-µl aliquot of the purified ligation reaction was loaded using a BioAnalyzer DNA 1000 LabChip. This purification step is recommended as the unpurified ligation reaction contains high amounts of salt and PEG that will inhibit the sample from running properly on the BioAnalyzer.
3. The remainder of the ligation reaction (190 µL) was used for gel isolation in Step 4.

### Step 3a: Microcon Filtration and Adaptor Construction. Total preparation time was approximately 25 min.

The Universal Adaptor ligation reaction requires a 100-fold excess of adaptors. To aid in the removal of these excess adaptors, the double-stranded gDNA library is filtered through a Microcon YM-100 filter device. Microcon YM-100 membranes can be used to remove double stranded DNA smaller than 125 bp. Therefore, unbound adaptors (44 bp), as well as adaptor dimers (88 bp) can be removed from the ligated gDNA library population. The following filtration protocol was used:
1. The 190 µL of the ligation reaction from Step 4 was applied into an assembled Microcon YM-100 device.
2. The device was placed in a centrifuge and spun at 5000 x g for approximately 6 minutes, or until membrane was almost dry.
3. To wash, 200 µl of 1X TE was added.
4. Sample was spun at 5000 x g for an additional 9 minutes, or until membrane was almost dry.
5. To recover, the reservoir was inserted into a new vial and spun at 3000 x g for 3 minutes. The reservoir was discarded. The recovered volume was approximately 10 µl. Next, 80 µl TE was added.

The Adaptors (A and B) were HPLC-purified and modified with phosphorothioate linkages prior to use. For Adaptor "A" (10 µM, 10 µl of 100 µM Adaptor A (44 bp, sense) was mixed with 10 µl of 100 µM Adaptor A (40 bp, antisense), and 30 µl of 1X Annealing Buffer (V_{f} = 50 µl) were mixed. The primers were annealed using the ANNEAL program on the Sample Prep Labthermal cycler (see below). For Adaptor "B" (10 µM, 10 µl of 100 µM Adaptor B (40 bp, sense) was mixed with 10 µl of 100 µM Adaptor B (44 bp, antisense), and 30 µl of 1X Annealing Buffer (V_{f}= 50 µl). The primers were annealed using the ANNEAL program on the Sample Prep Lab thermal cycler. Adaptor sets could be stored at -20°C until use.

ANNEAL-A program for primer annealing:
1.. Incubate at 95°C, 1 min;
2.. Decrease temperature to 15°C, at 0.1 °C/sec; and
3.. Hold at 15°C.

There was no orientation required for the genomic DNA insert fragment and the adaptors. Fragments could be ligated at either end. Four single-stranded DNA oligonucleotides were included in the Universal Adaptor set. Each single-stranded oligonucleotide was synthesized at 1 micromole scale and HPLC-purified. Each single-stranded oligonucleotide included four phosphorothioate linkages at each end.

### Step 4: Gel Electrophoresis and Extraction of Adapted DNA Library

The Universal Adaptor ligation protocol produces the following: 1) fragmented DNAs with adaptors on either end; 2) unbound single adaptors; or 3) the formation of adaptor dimers. Agarose gel electrophoresis is used as a method to separate and isolate the adapted DNA library population from the unligated, single adaptors and adaptor dimer populations. The procedure of DNase I digestion of genomic DNA yields a library population that ranges from 50-700 bp (Step 1). The addition of the 88-bp Universal Adaptor set will shift the population to a larger size and will result in a migration profile in the size range of approximately 130-800 bp. Adaptor dimers will migrate at 88 bp and adaptors unligated will migrate at 44 bp. Therefore, genomic DNA libraries in size ranges > 200 bp can be physically isolated from the agarose gel and purified using standard gel extraction techniques. Gel isolation of the adapted DNA library will result in the recovery of a library population in a size range that is ≥ 200 bp (size range of library can be varied depending on application). The following electrophoresis and extraction protocol was used.
1. A 2% agarose gel was prepared.
2. 10 µl of 10X Ready-Load Dye was added to the remaining 90 µl of the DNA ligation mixture.
3. The dye/ligation reaction mixture was loaded into the gel using four adjacent lanes (25 µl per lane).
4. 10 µl of the 100 bp ladder (0.1 µg/µl) was loaded two lanes away from ligation reaction lanes.
5. The gel was run at 100V for 3 hours.
6. When the gel run was complete, the gel was removed from the gel box and transferred to a flat surface covered with plastic wrap. DNA bands were visualized using a hand-held long-wave UV light. Using a sterile, single-use scalpel, the fragment sizes of 200 - 400 bp were cut out from the agarose gel. Using this approach, libraries with any size range can be isolated. It is also possible to isolate more than one size range. Where the library size range is 200-900 bp, it is possible to isolate several size ranges from a single well (i.e., 200-400 bp and 500-700 bp).
7. The DNA embedded in the agarose gel was isolated using a Qiagen MinElute Gel Extraction kit following the manufacturer's instructions. Briefly, Buffer QG was added to cover the agarose in the tube. The agarose was allowed to completely dissolve. The color of the Buffer QG was maintained by adjusting the pH according to the Qiagen instructions to minimize sample loss. Two MinElute spin columns (Qiagen) were used for purification. The large volume of dissolved agarose required each column to be loaded several times. The columns were eluded with 10 µl of Buffer EB which was pre-warmed at 55°C. The eluates were pooled to produce 20 µl of gDNA library.
8. One 1 µL aliquot of each isolated DNA library was analyzed using a BioAnalyzer DNA 1000 LabChip to assess the exact distribution of the DNA library population.

### Step 5 : Strand Displacement and Extension of Nicked Double Stranded DNA Library

Because the DNA oligonucleotides used for the Universal Adaptors are not phosphorylated, gaps are present at the 3' junctions of the fragmented gDNAs. These two "gaps" or "nicks" can be filled in by using a strand displacing DNA polymerase. The polymerase recognizes nicks, displaces the nicked strands, and extends the strand in a manner that results in repair of nicks and in the formation of non-nicked double-stranded DNA. The strand displacing enzyme used is the large fragment of *Bst* DNA polymerase.
1. In a 0.2 ml tube, 19 µl gel-extracted DNA library, 40 µl nH₂O, 8 µl 10X ThermoPol Reaction Buffer, 8 µl BSA (1 mg/ml), 2 µl dNTPs (10 mM), and 3 µl *Bst* I Polymerase (8 U/µl) were added in order.
2. The samples were mixed well and placed in a thermal cycler and incubated using the Strand Displacement incubation program: "BST". BST program for stand displacement and extension of nicked double-stranded DNA:
   1. Incubate at 65° C, 30 minutes;
   2. Incubate at 80° C, 10 minutes;
   3. Incubate at 58° C, 10 minutes; and
   4. Hold at 14° C.
3. One 1 µL aliquot of the *Bst*-treated DNA library was run using a BioAnalyzer DNA 1000 LabChip.

### Step 6: Preparation of Streptavidin Beads

Following the generation of unnicked double-stranded genomic DNA, it is necessary to isolate single-stranded genomic DNAs containing flanking Universal Adaptor sequences. This step outlines the binding of biotin-tagged double-stranded DNA to streptavidin beads. For preparing streptavidin beads, the following protocol was used.
1. 100 µl Dynal M-270 Streptavidin beads were washed two times with 200 µl of 1X Binding Buffer (1 M NaCl, 0.5 mM EDTA, 5 mM Tris, pH 7.5) by applying the magnetic beads to the MPC.
2. The beads were resuspended in 100 µl 2X Binding buffer, then the remaining 79 µl of the Bst-treated DNA sample (from Step 5) and 20 µl water was added.
3. The bead solution was mixed well and placed on a tube rotator at RT for 20 minutes. The bead mixtures were washed, using the MPC, two times with 100 µl of 1X Binding Buffer, then washed two times with nH₂O. Binding & Washing (B&W) Buffer (2X and 1X): 2X B&W buffer was prepared by mixing 10 mM Tris**·**HCl (pH 7.5), 1 mM EDTA, and 2 M NaCl. The reagents were combined as listed above and mixed thoroughly. The solution can be stored at RT for 6 months; 1X B&W buffer was prepared by mixing 2X B&W buffer with nH₂O, 1:1. The final concentrations were half the above, i.e., 5 mM Tris·HCl (pH 7.5), 0.5 mM EDTA, and 1M NaCl.

### Step 7: Isolation of single-stranded DNA Library using Streptavidin Beads

Following binding of the double-stranded gDNA library to streptavidin beads, it is preferred to isolate from the ligated pool only the single-stranded gDNAs containing Universal Adaptor A and Universal Adaptor B (desired populations are designated below with asterisks). Double-stranded genomic DNA fragment pools will have adaptors bound in the following possible configurations:
Universal Adaptor A- gDNA Fragment -Universal Adaptor A
Universal Adaptor B- gDNA Fragment -Universal Adaptor A*
Universal Adaptor A- gDNA Fragment -Universal Adaptor B*
Universal Adaptor B- gDNA Fragment -Universal Adaptor B

Because only the Universal Adaptor B has a 5' biotin moiety, magnetic streptavidin-containing beads can be used to bind all gDNA library species that possess the Universal Adaptor B. Genomic library populations that contain two Universal Adaptor A species (or nonligated species) do not bind to streptavidin-containing beads and are removed during the wash procedure. The species that remain bound to bead after washing include those with Universal Adaptors A and B or those with two Universal Adaptor B ends.

Genomic DNA species with two Universal Adaptor B sequences with two biotin molecules can bind to the streptavidin-containing beads at both ends. Species with A and B adaptors having only a single biotin molecule can bind to the beads only at the "B" end. To isolate the single-stranded population, the bead-bound double-stranded DNA is treated with a sodium hydroxide solution that serves to disrupt the hydrogen bonding between the complementary DNA strands. If the DNA fragment has biotin on each end (Universal Adaptor B ends), both resulting single strands remain bound to the beads. If the fragment has only a single biotin (Universal Adaptors A and B), then the complementary strand separates from the DNA-bead complex.

The resulting single-stranded genomic DNA library is collected from the solution phase and is quantitated, e.g., using pyrophosphate sequencing (PyroSequence) or by using a RNA Pico 6000 LabChip (Agilent, Palo Alto, CA). Single-stranded genomic DNA libraries are quantitated by calculating the number of molecules per unit volume. Single-stranded gDNA molecules are then annealed (at a half copy per bead to obtain one effective copy per bead) to 25-30 µm sepharose beads containing DNA capture primers (PCR primer B). The templates are then amplified using emulsion polymerase chain reaction protocols. Subsequent sequencing may be conducted using known techniques. For isolation of the single stranded library, the following protocol was used.
1. 250 µl Melt Solution (0.125 M NaOH, 0.1 M NaCl)was added to washed beads from Step 6 above.
2. The bead solution was mixed well and the bead mixture was incubated at room temperature for 10 minutes on a tube rotator.
3. A Dynal MPC (magnetic particle concentrator) was used, the pellet beads were carefully removed, and the supernatant was set aside. The 250-µl supernatant included the single-stranded DNA library.
4. In a separate tube, 1250 µl PB (from QiaQuick Purification kit) was added and the solution was neutralized by adding 9 µl of 20% acetic acid.
5. Using a Dynal MPC, beads from the 250-µl supernatant including the single-stranded gDNA library were pelleted and the supernatant was carefully removed and transferred to the freshly prepared PB/acetic acid solution.
6. The 1500 µl solution was purified using a single QiaQuick purification spin column (load sample through same column two times at 750 µl per load). The single-stranded DNA library was eluted with 50 µl EB.

Step 8a: Single-stranded gDNA Quantitation using Pyrophosphate Sequencing. Total preparation time was approximately 1 hr.
1. In a 0.2 ml tube, the following reagents were added in order:
   25 µl single-stranded gDNA
   1 µl MMP2B sequencing primer
   14 µl Library Annealing Buffer
   40 µl total
2. The DNA was allowed to anneal using the ANNEAL-S Program (see Appendix, below).
3. The samples were run on PSQ (pyrophosphate sequencing jig) to determine the number of picomoles of template in each sample (see below). Methods of sequencing can be found in U.S. Patent 6,274,320; U.S. Patent 4,863,849; U.S. Patent 6,210,891; and U.S. Patent 6,258,568, the disclosures of which are incorporated *in toto* herein by reference. Calculations were performed to determine the number of single-stranded gDNA template molecules per microliter. The remaining 25 µL of prepared single-stranded gDNA library was used for amplification and subsequent sequencing (approximately 1 x 10⁶ reactions).

### Step 8b: Single-stranded gDNA Quantitation using RNA Pico 6000 LabChip. Total preparation time was approximately 30 minutes.

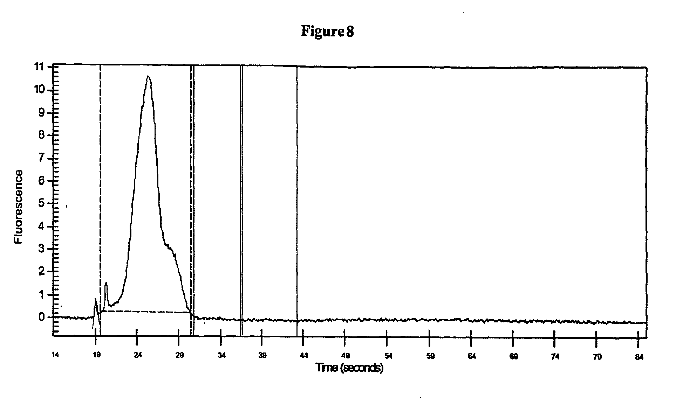

1. The mRNA Pico assay option was selected on the BioAnalyzer (Software version 2.12).
2. An RNA Pico 6000 LabChip was prepared on the BioAnalyzer according to the manufacturers' guidelines.
3. An RNA LabChip ladder (RNA 6000 ladder) was prepared according to manufacturer's (Ambion) directions. Briefly, the RNA LabChip ladder, in solution, was heated to 70°C for 2 minutes. The solution was chilled on ice for 5 minutes to snap cool the ladder. The solution was briefly centrifuged to clear any condensate from tube walls. The RNA LabChip Ladder was stored on ice and used within one day.
4. The ssDNA library to be analyzed was run in triplicate, in adjacent lanes, using three 1 µl aliquots.
5. The BioAnalyzer software was used to calculate the concentration of each ssDNA library lane (see the Table below and Figure 8. The average of all three lanes was used to calculate the DNA concentration of the library using the procedure outlined below.
   a. The peak integration lower limit line (large dash in Figure 8) was moved immediately in front of the library peak (see below).
   b. The peak integration upper limit line (large dash in the Figure 8) was moved immediately after the library peak. In this way, the peak integration line connecting the lower and upper integration lines followed the slope of the background.
   c. The mouse arrow was used to determine the average size of the peak in bases (usually near the peaks highest point) or a defined peak was used as chosen by the software.
   d. The integrated value was used for the amount of material in the peak.

The value obtained for picograms recovered was converted into molecules recovered (see Table, below). The library concentration was then determined (molecules per microliter).

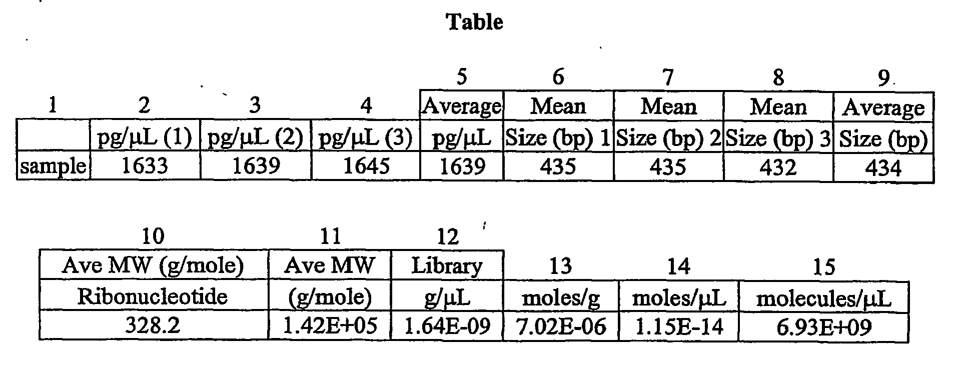

As shown in the Table above, the concentration of Library 1 was calculated as 1639 pg/µl (Column 5) and the average fragment size was 434 nucleotides (Column 9). These values were obtained from the Agilent 2100 software as described in Steps (a)-(d), above. The average molecular weight (MW) of a ribonucleotide is 328.2 g/mole (Column 10). The MW of the average library fragment (1.42 x 10⁵ g/mole, Column 11) was calculated by multiplying the average fragment length (434) by the average ribonucleotide (328.2). The quantitated library (1639 pg/µl) was converted to grams per microliter (1.64 x 10⁻⁹ g/µl, Column 12). The number of moles per microliter (1.15 x 10⁻¹⁴ moles/µl, Column 14) was calculated by dividing the grams per microliter (1.64 x 10⁻⁹ g/µl, Column 12) by the average molecular weight of the library fragments (1.42 x 10⁵, Column 11). Finally, the number of molecules per microliter (6.93 x 10⁹ molecules/µl, Column 15) was derived by multiplying the number of moles per microliter (1.15 x 10⁻¹⁴ moles/µl, Column 14) by Avogadro's number (6.02 x 10²³ molecules/mole).

The final library concentration was expected to be greater than 1 x 10⁸ molecules/µl. A more important factor for library quality was adaptor dimer concentration. In Figure 8, the height of the library peak was determined approximately 10 fold greater than the adaptor dimer peak (the first peak after the marker). A library of good quality is expected to have a peak height at least 2 fold greater than the dimer peak. It should be noted that the RNA Pico 6000 LabChip provided estimates within 500% accuracy of the single-stranded gDNA concentration. Thus, it was important to perform an initial sequencing run using a titration of template to determine the number of copies per bead (cpb) of input gDNA. The recommended input DNA is 2.5 cpb, 1 cpb, 0.5 cpb, and 0.1 cpb. This titration was easily checked using the 4slot bead loading chamber on a 14 x 43 PTP.

### Step 9: Dilution and Storage of Single-Stranded gDNA library

The single-stranded gDNA library was eluted and quantitated in Buffer EB. To prevent degradation, the single-stranded gDNA library was stored frozen at -20°C in the presence of EDTA. After quantitation, an equal volume of 10 mM TE was added to the library stock. All subsequent dilutions was in TE. The yield was as follows:
Remaining final volume of ssDNA library following PSQ analysis = 25 µl.
Remaining final volume of ssDNA library following LabChip analysis = 47 µl.

For the initial stock dilution, single-stranded gDNA library was diluted to 100 million molecules/µl in 1X Library-Grade Elution Buffer. Aliquots of single-stranded gDNA library were prepared for common use. For this, 200,000 molecules/µl were diluted in 1X Library-Grade Elution Buffer and 20 µl aliquots were measured. Single-use library aliquots were stored at 20°C.

### Step 10: Emulsion Polymerase Chain Reaction.

Where increased numbers of cpb were preferred, bead emulsion PCR was performed as described in International Patent Application Publication No. WO2004/069849, filed June 6 2003, incorporated herein by reference in its entirety.

### Reagent preparation

The Stop Solution (50 mM EDTA) included 100 µl of 0.5 M EDTA mixed with 900 µl of nH₂O to obtain 1.0 ml of 50 mM EDTA solution. For 10 mM dNTPs, (10 µl dCTP (100 mM), 10 µl dATP (100 mM), 10 µl dGTP (100 mM), and 10 µl dTTP (100 mM) were mixed with 60 µl molecular biology grade water. All four 100 mM nucleotide stocks were thawed on ice. Then, 10 µl of each nucleotide was combined with 60 µl of nH₂O to a final volume of 100 µl, and mixed thoroughly. Next, 1 ml aliquots were dispensed into 1.5 ml microcentrifuge tubes. The stock solutions could be stored at 20°C for one year.

The 10 X Annealing buffer included 200 mM Tris (pH 7.5) and 50 mM magnesium acetate. For this solution, 24.23 g Tris was added to 800 ml nH₂O and the mixture was adjusted to pH 7.5. To this solution, 10.72 g of magnesium acetate was added and dissolved completely. The solution was brought up to a final volume of 1000 ml and could be stored at 4°C for 1 month. The 10 X TE included 100 mM Tris·HCl (pH 7.5) and 50 mM EDTA. These reagents were added together and mixed thoroughly. The solution could be stored at room temperature for 6 months.

### Example 2: Primer Design

As discussed above, the universal adaptors are designed to include: 1) a set of unique PCR priming regions that are typically 20 bp in length (located adjacent to (2)); 2) a set of unique sequencing priming regions that are typically 20 bp in length; and 3) optionally followed by a unique discriminating key sequence consisting of at least one of each of the four deoxyribonucleotides (i.e., A, C, G, T). The probability of cross-hybridization between primers and unintended regions of the genome of interest is increased as the genome size increases and length of a perfect match with the primer decreases. However, this potential interaction with a cross-hybridizing region (CHR) is not expected to produce problems for the reasons set forth below.

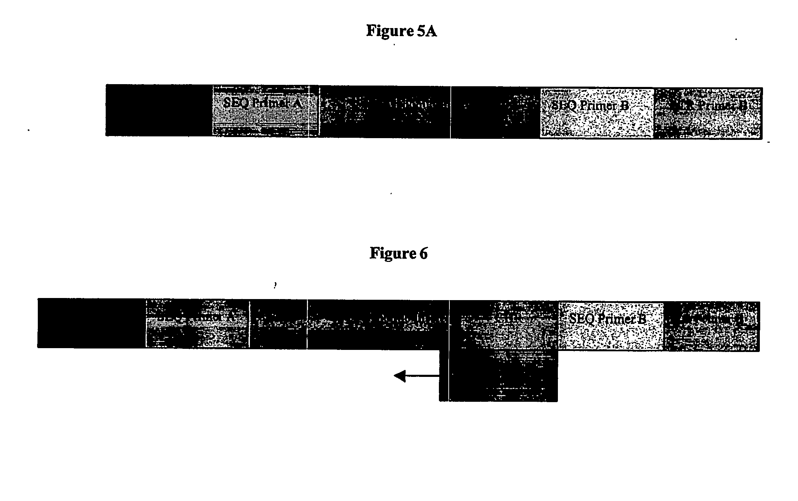

In a preferred embodiment of the present invention, the single-stranded DNA library is utilized for PCR amplification and subsequent sequencing. Sequencing methodology requires random digestion of a given genome into 150 to 500 base pair fragments, after which two unique bipartite primers (composed of both a PCR and sequencing region) are ligated onto the 5' and 3' ends of the fragments (Figure 5). Unlike typical PCR amplifications where an existing section of the genome is chosen as a priming site based on melting temperature (Tₘ), uniqueness of the priming sequence within the genome and proximity to the particular region or gene of interest, the disclosed process utilizes synthetic priming sites that necessitates careful *de novo* primer design.

### Tetramer Selection:

Strategies for *de novo* primer design are found in the published literature regarding work conducted on molecular tags for hybridization experiments (see, Hensel, M. and D.W. Holden, Molecular genetic approaches for the study of virulence in both pathogenic bacteria and fungi. Microbiology, 1996. 142(Pt 5): p. 1049-58; Shoemaker, D.D., et al., Quantitative phenotypic analysis of yeast deletion mutants using a highly parallel molecular bar-coding strategy. Nat Genet, 1996. 14(4): p. 450-6) and PCR/LDR (polymerase chain reaction/ligation detection reaction) hybridization primers (see, Gerry, N.P., et al., Universal DNA microarray method for multiplex detection of low abundance point mutations. Journal of Molecular Biology, 1999. 292: p. 251-262; Witowski, N.E., et al., Microarray-based detection of select cardiovascular disease markers. BioTechniques, 2000. 29(5): p. 936-944.).

The PCR/LDR work was particularly relevant and focused on designing oligonucleotide "zipcodes", 24 base primers comprised of six specifically designed tetramers with a similar final Tₘ. (see, Gerry, N.P., et al., Universal DNA microarray method for multiplex detection of low abundance point mutations. Journal of Molecular Biology, 1999. 292: p. 251-262; U.S. Pat, No. 6,506,594). Tetrameric components were chosen based on the following criteria: each tetramer differed from the others by at least two bases, tetramers that induced self-pairing or hairpin formations were excluded, and palindromic (AGCT) or repetitive tetramers (TATA) were omitted as well. Thirhy-six of the 256 (4⁴) possible permutations met the necessary requirements and were then subjected to further restrictions required for acceptable PCR primer design (Table 1).

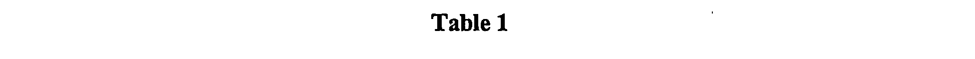

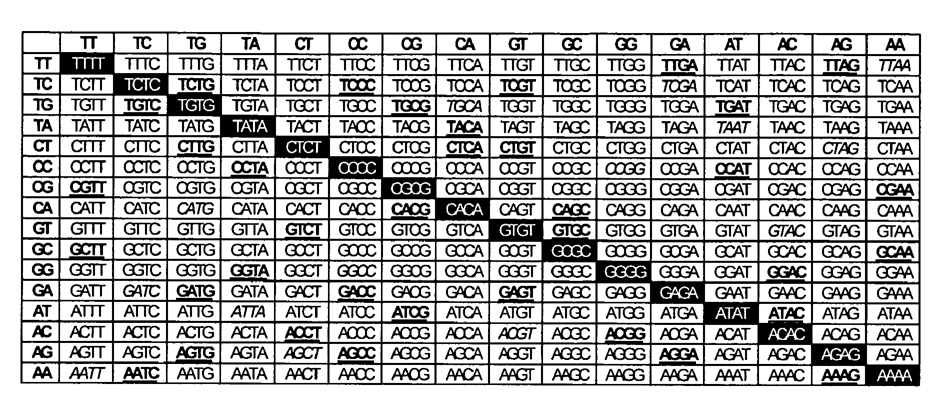

Table 1 shows a matrix demonstrating tetrameric primer component selection based on criteria outlined by Gerry et al. 1999. J. Mol. Bio. 292: 251-262. Each tetramer was required to differ from all others by at least two bases. The tetramers could not be palindromic or complimentary with any other tetramer. Thirty-six tetramers were selected (bold, underlined); italicized sequences signal palindromic tetramers that were excluded from consideration.

### Primer Design:

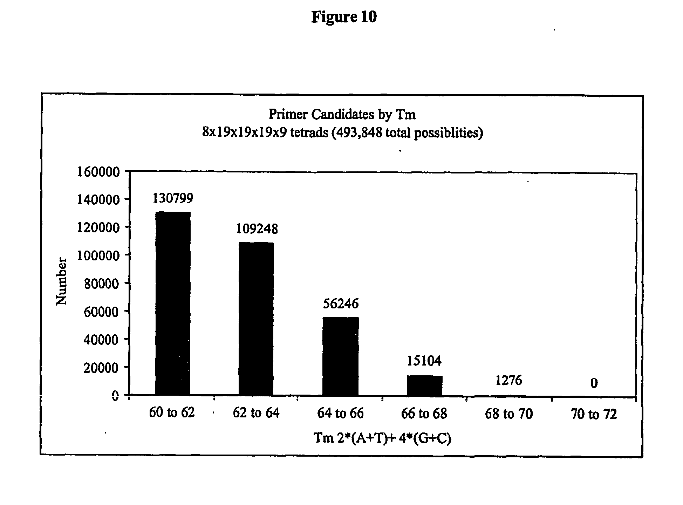

The PCR primers were designed to meet specifications common to general primer design (see, Rubin, E. and A.A. Levy, A mathematical model and a computerized simulation of PCR using complex templates. Nucleic Acids Res, 1996. 24(18): p. 3538-45; Buck, G.A., et al., Design strategies and performance of custom DNA sequencing primers. Biotechniques, 1999. 27(3): p. 528-36), and the actual selection was conducted by a computer program, MMP. Primers were limited to a length of 20 bases (5 tetramers) for efficient synthesis of the total bipartite PCR/sequencing primer. Each primer contained a two base GC clamp on the 5' end, and a single GC clamp on the 3' end (Table 2), and all primers shared similar Tₘ (+/-2°C) (Figure 10). No hairpinning within the primer (internal hairpin stem ΔG > -1.9 kcal/mol) was permitted. Dimerization was also controlled; a 3 base maximum acceptable dimer was allowed, but it could occur in final six 3' bases, and the maximum allowable ΔG for a 3' dimer was -2.0 kcal/mol. Additionally, a penalty was applied to primers in which the 3' ends were too similar to others in the group, thus preventing cross- hybridization between one primer and the reverse complement of another.

**Table 2**

| | **1-pos** | **2-pos** | **3-pos** | **4-pos** | **5-pos** |
|---|---|---|---|---|---|
| 1 | CCAT | TGAT | TGAT | TGAT | ATAC |
| 2 | CCTA | CTCA | CTCA | CTCA | AAAG |
| 3 | CGAA | TACA | TACA | TACA | TTAG |
| 4 | CGTT | AGCC | AGCC | AGCC | AATC |
| 5 | GCAA | GACC | GACC | GACC | TGTC |
| 6 | GCTT | TCCC | TCCC | TCCC | AGTG |
| 7 | GGAC | ATCG | ATCG | ATCG | CTTG |
| 8 | GGTA | CACG | CACG | CACG | GATG |
| 9 | | TGCG | TGCG | TGCG | TCTG |
| 10 | | ACCT | ACCT | ACCT | |
| 11 | | GTCT | GTCT | GTCT | |
| 12 | | AGGA | AGGA | AGGA | |
| 13 | | TTGA | TTGA | TTGA | |
| 14 | | CAGC | CAGC | CAGC | |
| 15 | | GTGC | GTGC | GTGC | |
| 16 | | ACGG | ACGG | ACGG | |
| 17 | | CTGT | CTGT | CTGT | |
| 18 | | GAGT | GAGT | GAGT | |
| 19 | | TCGT | TCGT | TCGT | |

Table 2 shows possible permutations of the 36 selected tetrads providing two 5' and a single 3' G/C clamp. The internal positions are composed of remaining tetrads. This results in 8 x 19 x 19 x 19 x 9 permutations, or 493,848 possible combinations. Figure 10 shows first pass, Tₘ based selection of acceptable primers, reducing field of 493,848 primers to 56,246 candidates with Tₘ of 64 to 66°C.

**Table 3**

| The probability of perfect sequence matches for primers increases with decreasing match length requirements and increasing size of the genome of interest. | | | | |
|---|---|---|---|---|
| Match Length | Perfect match probability (1/(4^length)) | % chance for match in Adeno ~ 35K bases | % chance for match in NCBI bacterial dabase~488M bases | %chance for match in Human -3B bases |
| 20 | 9.1E-13 | 0.00% | 0.04% | 0.27% |
| 19 | 7.3E-12 | 0.00% | 0.65% | 4.32% |
| 18 | 4.4E-11 | 0.00% | 5.76% | 34.37% |
| 17 | 23E-10 | 0.00% | 35.69% | 99.17% |
| 16 | 12E-09 | 0.02% | 97.52% | > 100% |
| 15 | 5.6E-09 | 0.12% | > 100% | >100% |
| 14 | 26E-08 | 0.64% | > 100% | >100% |
| 13 | 1.2E-07 | 3.29% | > 100% | > 100% |
| 12 | 5.4E-07 | 15.68% | > 100% | > 100% |
| 11 | 24E-06 | 58.16% | > 100% | > 100% |
| 10 | 1.0E-05 | 99.35% | > 100% | > 100% |
| 9 | 4.6E-05 | 99.77% | > 100% | > 100% |
| 8 | 20E-04 | > 100% | > 100% | > 100% |
| 7 | 8.5E-04 | > 100% | > 100% | > 100% |
| 6 | 3.7E-03 | > 100% | > 100% | > 100% |
| 5 | 1.6E-02 | > 100% | > 100% | > 100% |
| 4 | 6.4E-02 | > 100% | > 100% | > 100% |
| 3 | 25E-01 | > 100% | >100% | > 100% |
| 2 | 7.1E-01 | > 100% | >100% | > 100% |
| 1 | 1.0E+00 | > 100% | > 100% | > 100% |

The possibility of complimentary regions occurring within the genome of interest was not a major concern in the primer design process despite the reported tolerance of PCR to mismatches in complex sample populations (see, e.g., Rubin, E. and A.A. Levy, A mathematical model and a computerized simulation of PCR using complex templates. Nucleic Acids Res, 1996. 24(18): p. 3538-45). Although the probability of finding a perfect match to a 20 base primer is extremely low (4²⁰) (Table 3), the probability of finding less non-consecutive matches increases significantly with the size of the genome of interest. As a result, the probability of finding a perfect match of at least 10 of 20 bases is 99.35% for an Adenovirus genome. The probability of finding a 16 base perfect match is 97% for the sequences in the NCBI database (approximately 100 times larger than the Adenovirus genome). The probability of finding a 17 base perfect match to a 20 base primer is 99% for the sequences in the human genome (3 billion bases).

The high probability of primer cross-hybridization to regions of the genome is less problematic than one might expect due to the random DNA digestion used to produce the template fragments. Thus, the effects of a cross-hybridizing region (CHR) are fairly benign. It is unlikely that a CHR would be able to successfully compete with the perfect match between the PCR primers in solution and the template. In addition, any primers that include mismatches at their 3' end would be at a significant competitive disadvantage. Even if a CHR should out compete the intended PCR primer, it would produce a truncated PCR product, without a downstream site for the sequencing primer. If the truncated product could be driven to the capture bead and immobilized, one of two situations would result. If the CHR out-competed the solution-phase primer, then the immobilized product would lack a sequencing primer binding site, and would result in an empty PicoTiter plate (PTP) well. If the CHR out-competed the bead-bound primer, the sequencing primer would still be present, and the only effect would be a shorter insert. Neither result would unduly compromise the sequencing quality. Given the large amount of genomic material used in the sample preparation process (currently 25 µg, containing 5.29 x 10¹⁶ copies of the 35 Kb Adenovirus genome), oversampling can be used to provide fragments that lack the complete CHR, and allow standard PCR amplification of the region in question.

### Example 3: Sample Preparation by Nebulization

### Preparation of DNA by Nebulization

The purpose of the Nebulization step is to fragment a large stretch of DNA such as a whole genome or a large portion of a genome into smaller molecular species that are amenable to DNA sequencing. This population of smaller-sized DNA species generated from a single DNA template is referred to as a library. Nebulization shears double-stranded template DNA into fragments ranging from 50 to 900 base pairs. The sheared library contains single-stranded ends that are end-repaired by a combination of T4 DNA polymerase, *E. coli* DNA polymerase I (Klenow fragment), and T4 polynucleotide kinase. Both T4 and Klenow DNA polymerases are used to "fill-in" 3' recessed ends (5' overhangs) of DNA via their 5'-3' polymerase activity. The single-stranded 3'-5' exonuclease activity of T4 and Klenow polymerases will remove 3' overhang ends and the kinase activity of T4 polynucleotide kinase will add phosphates to 5' hydroxyl termini.

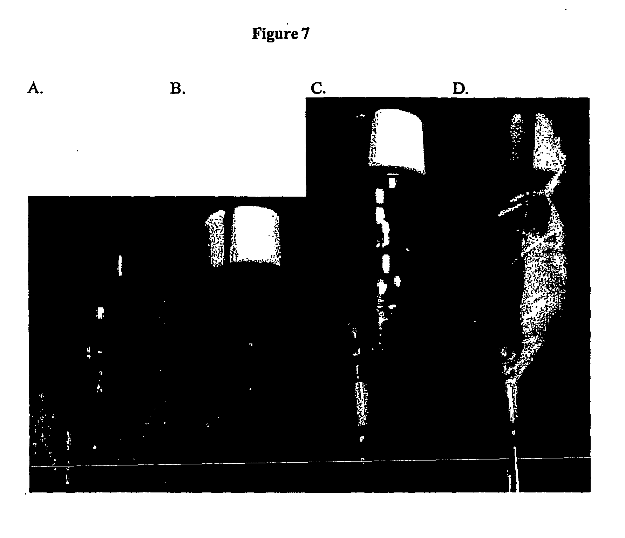

The sample was prepared as follows:
1. 15 µg of gDNA (genomic DNA) was obtained and adjusted to a final volume of 100 µl in 10 mM TE (10 mM Tris, 0.1 mM EDTA, pH 7.6; see reagent list at the end of section). The DNA was analyzed for contamination by measuring the O.D. _{260/280} ratio, which was 1.8 or higher. The final gDNA concentration was expected to be approximately 300 µg/ml.
2. 1600 ul of ice-cold Nebulization Buffer (see end of section) was added to the gDNA.
3. The reaction mixture was placed in an ice-cold nebulizer (CIS-US, Bedford, MA).
4. The cap from a 15 ml snap cap falcon tube was placed over the top of the nebulizer (Figure 7A).
5. The cap was secured with a clean Nebulizer Clamp assembly, consisting of the fitted cover (for the falcon tube lid) and two rubber O-rings (Figure 7B).
6. The bottom of the nebulizer was attached to a nitrogen supply and the entire device was wrapped in parafilm (Figures 7C and 7D).
7. While maintaining nebulizer upright (as shown in Figure 7D), 50 psi (pounds per square inch) of nitrogen was applied for 5 minutes. The bottom of the nebulizer was tapped on a hard surface every few seconds to force condensed liquid to the bottom.
8. Nitrogen was turned off after 5 minutes. After the pressure had normalized (30 seconds), the nitrogen source was remove from the nebulizer.
9. The parafilm was removed and the nebulizer top was unscrewed. The sample was removed and transferred to a 1.5 ml microcentrifuge tube.
10. The nebulizer top was reinstalled and the nebulizer was centrifuged at 500 rpm for 5 minutes.
11. The remainder of the sample in the nebulizer was collected. Total recovery was about 700 µl.
12. The recovered sample was purified using a QIAquick column (Qiagen Inc., Valencia, CA) according to manufacturer's directions. The large volume required the column to be loaded several times. The sample was eluted with 30 µl of Buffer EB (10 mM Tris HCl, pH 8.5;supplied in Qiagen kit) which was pre-warmed at 55°C.
13. The sample was quantitated by UV spectroscopy (2 µl in 198 µl water for 1:100 dilution).

### Enzymatic Polishing

Nebulization of DNA templates yields many fragments of DNA with frayed ends. These ends are made blunt and ready for ligation to adaptor fragments by using three enzymes, T4 DNA polymerase, *E. coli* DNA polymerase (Klenow fragment) and T4 polynucleotide kinase.

The sample was prepared as follows:
1. In a 0.2 ml tube the following reagents were added in order:
   28 µl purified, nebulized gDNA fragments
   5 µl water
   5 µl 10 X T4 DNA polymerase buffer
   5 µl BSA (1mg/ml)
   2 µl dNTPs (10 mM)
   5 µl T4 DNA polymerase (3 units/µl)
   50 µl final volume
2. The solution of step 1 was mixed well and incubated at 25°C for 10 minutes in a MJ thermocycler (any accurate incubator may be used).
3. 1.25 µl *E. coli* DNA polymerase (Klenow fragment) (5 units/ml) was added.
4. The reaction was mixed well and incubated in the MJ thermocycler for 10 minutes at 25°C and for an additional 2 hrs at 16°C.
5. The treated DNA was purified using a QiaQuick column and eluted with 30 µl of Buffer EB (10 mM Tris HCl, pH 8.5) which was pre-warmed at 55°C.
6. The following reagents were combined in a 0.2 ml tube:
   30 µl Qiagen purified, polished, nebulized gDNA fragments
   5 µl water
   5 µl 10 X T4 PNK buffer
   5 µl ATP (10 mM)
   5 µl T4 PNK (10 units/ml)
   50 µl final volume
7. The solution was mixed and placed in a MJ thermal cycler using the T4 PNK program for incubation at 37°C for 30 minutes, 65°C for 20 minutes, followed by storage at 14°C.
8. The sample was purified using a QiaQuick column and eluted in 30 µl of Buffer EB which was pre-warmed at 55°C.
10. A 2 µl aliquot of the final polishing reaction was held for analysis using a BioAnalyzer DNA 1000 LabChip (see below).

### Ligation of Adaptors

The procedure for ligating the adaptors was performed as follows:
1. In a 0.2 ml tube the following reagents were added in order:
   20.6 µl molecular biology grade water
   28 µl digested, polished gDNA Library
   60 µl 2 X Quick Ligase Reaction Buffer
   1.8 µl MMP (200 pmol/µl) Universal Adaptor set
   9.6 µl Quick Ligase
   120 µl total
   The above reaction was designed for 5 µg and was scaled depending on the amount of gDNA used.
2. The reagents were mixed well and incubated at 25°C for 20 minutes. The tube was on ice until the gel was prepared for agarose gel electrophoresis.

### Gel Electrophoresis and Extraction of Adapted gDNA Library

Nebulization of genomic DNA yields a library population that ranges from 50-900 bp. The addition of the 88-bp Universal Adaptor set will shift the population to a larger size and will result in a migration profile with a larger size range (approximately 130-980 bp). Adaptor dimers will migrate at 88 bp and adaptors not ligated will migrate at 44 bp. Therefore, genomic DNA libraries isolated in size ranges ≥250 bp can be physically isolated from the agarose gel and purified using standard gel extraction techniques. Gel isolation of the adapted gDNA library will result in the recovery of a library population in a size range that is ≥250 bp (size range of library can be varied depending on application). The library size range after ligation of adapters is 130 to 980 bp. It should be noted that the procedure may be adapted for isolation of any band size range, such as, for example, 130 to 200 bp, 200 to 400 bp, 250 to 500 bp, 300 to 600 bp, 500 to 700 bp and the like by cutting different regions of the gel. The procedure described below was used to isolated fragments of 250 bp to 500 bp.

A 150 ml agarose gel was prepared to include 2% agarose, 1X TBE, and 4.5 ul ethidium bromide (10 mg/ml stock). The ligated DNA was mixed with 10X Ready Load Dye and loaded onto the gel. In addition, 10 µl of a100-bp ladder (0.1 µg/µl) was loaded on two lanes away from the ligation reaction flanking the sample. The gel was electrophoresed at 100 V for 3 hours. When the gel run was complete, the gel was removed from the gel box, transferred to a GelDoc, and covered with plastic wrap. The DNA bands were visualized using the Prep UV light. A sterile, single-use scalpel, was used to cut out a library population from the agarose gel with fragment sizes of 250 - 500 bp. This process was done as quickly as possible to prevent nicking of DNA. The gel slices were placed in a 15 ml falcon tube. The agarose-embedded gDNA library was isolated using a Qiagen MinElute Gel Extraction kit. Aliquots of each isolated gDNA library were analyzed using a BioAnalyzer DNA 1000 LabChip to assess the exact distribution of the gDNA library population.

### Strand Displacement and Extension of the gDNA Library and Isolation of the Single Stranded gDNA Library Using Streptavidin Beads

Strand displacement and extension of nicked double-stranded gDNA library was performed as described in Example 1, with the exception that the *Bst*-treated samples were incubated in the thermal cycler at 65° C for 30 minutes and placed on ice until needed. Streptavidin beads were prepared as described in Example 1, except that the final wash was performed using two washes with 200 µl 1X Binding buffer and two washes with 200 µl nH₂O. Single-stranded gDNA library was isolated using streptavidin beads as follows. Water from the washed beads was removed and 250 µl of Melt Solution (see below) was added. The bead suspension was mixed well and incubated at room temperature for 10 minutes on a tube rotator. In a separate tube, 1250 µl of PB (from the QiaQuick Purification kit) and 9 µl of 20% acetic acid were mixed. The beads in 250 µl Melt Solution were pelleted using a Dynal MPC and the supernatant was carefully removed and transferred to the freshly prepared PB/acetic acid solution. DNA from the 1500 µl solution was purified using a single MinElute purification spin column. This was performed by loading the sample through the same column twice at 750 µl per load. The single stranded gDNA library was eluted with 15 µl of Buffer EB which was pre-warmed at 55°C.

### Single Strand gDNA Quantitation and Storage

Single-stranded gDNA was quantitated using RNA Pico 6000 LabChip as described in Example 1. In some cases, the single stranded library was quantitated by a second assay to ensure the initial Agilent 2100 quantitation was performed accurately. For this purpose, RiboGreen quantitation was performed as described (ssDNA Quantitation by Fluorometry) to confirm the Agilent 2100 quantitation. If the two estimates differed by more than 3 fold, each analysis was repeated. If the quantitation showed greater than a 3 fold difference between the two procedures, a broader range of template to bead was used.

Dilution and storage of the single stranded gDNA library was performed as described in Example 1. The yield was as follows:
Remaining final volume of ssDNA library following LabChip analysis =12 µl.
Remaining final volume of ssDNA library following RiboGreen analysis = 9 µl.
Final volume of ssDNA library after the addition of TE = 18 µl.

An equal volume of TE was added to single-stranded gDNA library stock. Single-stranded gDNA library to 1 x 10⁸ molecules/µl in Buffer TE. Stock was diluted (1/500) to 200,000 molecules/µl in TE and 20 µl aliquots were prepared.

### Library Fragment Size Distribution After Nebulization

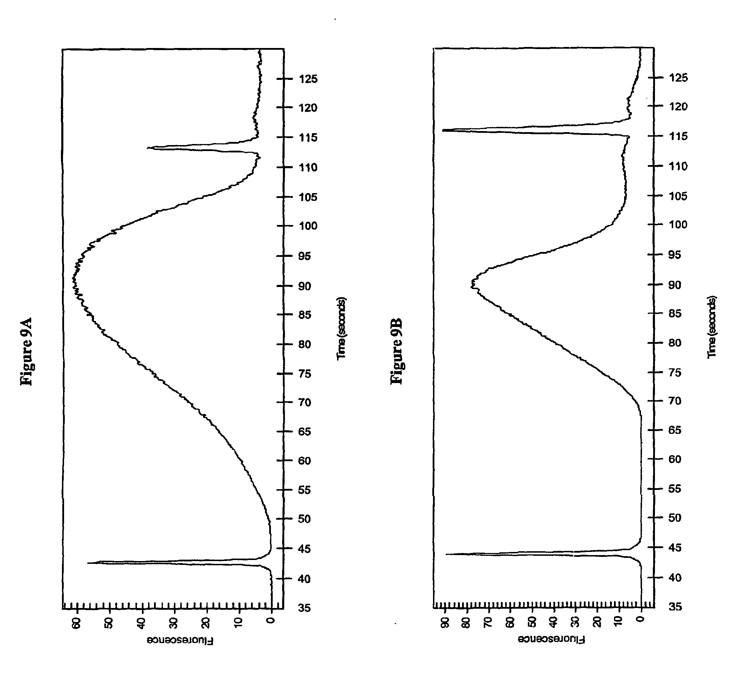

Typical results from Agilent 2100 DNA 1000 LabChip analysis of 1 µl of the material following Nebulization and polishing are shown in Figure 9A. The size range distribution of the majority of the product was expected to fall around 50 to 900 base pairs. The mean size (top of peak) was expected to be approximately 450 bp. Typical results from gel purification of adaptor ligated library fragments are shown in Figure 9B.

### Reagents

Unless otherwise specified, the reagents listed in the Examples represent standard reagents that are commercially available. For example, Klenow, T4 DNA polymerase, T4 DNA polymerase buffer, T4 PNK, T4 PNK buffer, Quick T4 DNA Ligase, Quick Ligation Buffer, *Bst* DNA polymerase (Large Fragment) and ThermoPol reaction buffer are available from New England Biolabs (Beverly, MA). dNTP mix is available from Pierce (Rockford, IL). Agarose, UltraPure TBE, BlueJuice gel loading buffer and Ready-Load 100bp DNA ladder may be purchased from Invitrogen (Carlsbad, CA). Ethidium Bromide and 2-Propanol may be purchased from Fisher (Hampton, NH). RNA Ladder may be purchased from Ambion (Austin, TX). Other reagents are either commonly known and/or are listed below:

### Melt Solution:

| **Ingredient** | **Quantity Required** | **Vendor** | **Stock Number** |
|---|---|---|---|
| NaCl (5 M) | 200 µl | Invitrogen | 24740-011 |
| NaOH (10 N) | 125 µl | Fisher | SS255-1 |
| molecular biology grade water | 9.675 ml | Eppendorf | 0032-006-205 |

The Melt Solution included 100 mM NaCl, and 125 mM NaOH. The listed reagents were combined and mixed thoroughly. The solution could be stored at RT for six months.

Binding & Washing (B&W) Buffer (2X and 1X):

| **Ingredient** | **Quantity Required** | **Vendor** | **Stock Number** |
|---|---|---|---|
| UltraPure Tris-HCl (pH 7.5, 1 M) | 250 µl | Invitrogen | 15567-027 |
| EDTA (0.5M) | 50 µl | Invitrogen | 15575-020 |
| NaCl (5 M) | 10 ml | Invitrogen | 24740-011 |
| molecular biology grade water | 14.7 ml | Eppendorf | 0032-006-205 |

The 2X B&W buffer included final concentrations of 10 mM Tris-HCl (pH 7.5), 1 mM EDTA, and 2 M NaCl. The listed reagents were combined by combined and mixed thoroughly. The solution could be stored at RT for 6 months. The 1X B&W buffer was prepared by mixing 2X B&W buffer with picopure H₂O, 1:1. The final concentrations was half of that listed the above, i.e., 5 mM Tris-HCl (pH 7.5), 0.5 mM EDTA, and 1 M NaCl.

Other buffers included the following. 1X T4 DNA Polymerase Buffer: 50 mM NaCl, 10 mM Tris-HCl, 10 mM MgCl2, 1 mM dithiothreitol (pH 7.9 @ 25°C). TE: 10 mM Tris, 1 mM EDTA.

Special Reagent preparation:
TE (10 mM):

| **Ingredient** | **Quantity Required** | **Vendor** | **Stock Number** |
|---|---|---|---|
| TE (1M) | 1 ml | Fisher | BP1338-1 |
| molecular biology grade water | 99 ml | Eppendorf | 0032-006-205 |

Reagents were mixed and the solution could be stored RT for six months.

Nebulization Buffer:

| **Ingredient** | **Quantity Required** | **Vendor** | **Stock Number** |
|---|---|---|---|
| Glycerol | 53.1 ml | Sigma | G5516 |
| molecular biology grade water | 42.1 ml | Eppendorf | 0032-006-205 |
| UltraPure Tris-HCl (pH 7.5, 1 M) | 3.7 ml | Invitrogen | 15567-027 |
| EDTA(0.5M) | 1.1 ml | Sigma | M-10228 |

All reagents were added (glycerol was added last) to a Stericup and mixed well. The solution was labeled and could be stored at RT for six months.

ATP (10 mM):

| **Ingredient** | **Quantity Required** | **Vendor** | **Stock Number** |
|---|---|---|---|
| ATP (100 mM) | 10 µl | Roche | 1140965 |
| molecular biology grade water | 90 µl | Eppendorf | 0032-006-205 |

The reagents were mixed and the solution could be stored at -20°C for six months.

BSA (1 mg/ml):

| **Ingredient** | **Quantity Required** | **Vendor** | **Stock Number** |
|---|---|---|---|
| BSA (10 mg/ml) | 10 µl | NEB | M0203 kit |
| Molecular Biology Grade water | 90 µl | Eppendorf | 0032-006-205 |

The reagents were mixed and the solution could be stored at 4°C for six months.

Library Annealing buffer, 10X:

| **Ingredient** | **Quantity Req.** | **Vendor** | **Stock No.** |
|---|---|---|---|
| UltraPure Tris-HCl (pH 7.5, 1 M) | 200 ml | Invitrogen | 15567-027 |
| Magnesium acetate, enzyme grade (1 M) | 10.72 g | Fisher | BP-215-500 |
| Molecular Biology Grade water | ~ 1L | Eppendorf | 0032-006-205 |

The 10 X Annealing Buffer included 200 mM Tris (pH 7.5) and 50 mM magnesium acetate. For this buffer, 200 ml of Tris was added to 500 ml picopure H₂O. Next, 10.72 g of magnesium acetate was added to the solution and dissolved completely. The solution was adjusted to a final volume of 1000 ml. The solution could be stored at 4°C for six months. To avoid the potential for contamination of libraries, the buffer was aliquotted for single or short-term usage.

Adaptors:
Adaptor "A" (400 µM):

| **Ingredient** | **Quantity Req.** | **Vendor** | **Stock No.** |
|---|---|---|---|
| Adaptor A (sense; HPLC-purified, phosphorothioate linkages, 44 bp, 1000 pmol/µl) | 10.0 µl | IDT | custom |
| Adaptor A (antisense; HPLC-purified, Phosphorothioate linkages, 40 bp, 1000 pmol/µl) | 10.0 µl | IDT | custom |
| Annealing buffer (10X) | 2.5 µl | 454 Corp. | previous table |
| molecular biology grade water | 2.5 µl | Eppendorf | 0032-006-205 |

For this solution, 10 µl of 1000 pmol/µl Adaptor A (44 bp, sense) was mixed with 10 µl of 1000 pmol/µl Adaptor A (40 bp, antisense), 2.5 µl of 10X Library Annealing Buffer, and 2.5 µl of water (V_{f}= 25 µl). The adaptors were annealed using the ANNEAL-A program (see Appendix, below) on the Sample Prep Lab thermal cycler. More details on adaptor design are provided in the Appendix.

Adaptor "B" (400 µM):

| **Ingredient** | **Quantity Req.** | **Vendor** | **Stock No.** |
|---|---|---|---|
| Adaptor B (sense; HPLC-purified, phosphorothioate linkages, 40 bp,1000 pmol/µl)) | 10 µl | IDT | Custom |
| Adaptor B (anti; HPLC-purified, phosphorothioate linkages, 5'Biotinylated, 44 bp,1000 pmol/µl) | 10 µl | IDT | Custom |
| Annealing buffer (10X) | 2.5 µl | 454 Corp. | previous table |
| molecular biology grade water | 2.5 µl | Eppendorf | 0032-006-205 |

For this solution, 10 µl of 1000 pmol/µl Adaptor B (40 bp, sense) was mixed with 10 µl of 1000 pmol/µl Adaptor B (44 bp, anti), 2.5 µl of 10 X Library Annealing Buffer, and 2.5 µl of water (V_{f} = 25 µl). The adaptors were annealed using the ANNEAL-A program (see Appendix) on the Sample Prep Lab thermal cycler. After annealing, adaptor "A" and adaptor "B" (V_{f}= 50 µl) were combined. Adaptor sets could be stored at -20°C until use.

20% Acetic Acid:

| **Ingredient** | **Quantity Required** | **Vendor** | **Stock Number** |
|---|---|---|---|
| acetic acid, glacial | 2 ml | Fisher | A35-500 |
| molecular biology grade water | 8 ml | Eppendorf | 0032-006-205 |

For this solution, glacial acetic acid was added to the water.. The solution could be stored at RT for six months.

Appendix
Adaptor Annealing Program:
ANNEAL-A program for primer annealing:
1. Incubate at 95°C, 1 min;
2. Reduce temperature to 15°C at 0.1°C/sec; and
3. Hold at 14°C.

T4 Polymerase/Klenow POLISH program for end repair:
1. Incubate at 25°C, 10 minutes;
2. Incubate at 16°C, 2 hours; and
3. Hold at 4°C.

T4 PNK Program for end repair:
1. Incubate at 37°C, 30 minutes;

2.	Incubate at 65°C, 20 minutes; and
3.	Hold at 14°C.

BST program for stand displacement and extension of nicked double-stranded gDNA:
1.	Incubate at 65° C, 30 minutes; and
2.	Hold at 14° C.

### References

Hamilton, S.C., J.W. Farchaus and M.C. Davis. 2001. DNA polymerases as engines for biotechnology. BioTechniques 31:370.
QiaQuick Spin Handbook (QIAGEN, 2001): hypertext transfer protocol://world wide web.qiagen.com/literature/bandbooks/qqspin/1016893HBQQSpin_PCR_mc_prot.pdf.
Quick Ligation Kit (NEB): hypertext transfer protocol://world wide web.neb.com/neb/products/mod_enzymes/M2200.html.
MinElute kit (QIAGEN): hypertext transfer protocol://world wide web.qiagen.coml/iterature/handbooks/minelute/1016839_HBMinElute_Prot_Gel.pdf.
Biomagnetic Techniques in Molecular Biology, Technical Handbook, 3rd edition (Dynal, 1998): hypertext transfer protocol://world wide web.dynal.no/kunder/dynal/DynalPub36.nsf/cb927fbab127a0ad4125683b004b011c/4908f5b 1a665858a41256adf005779f2/$FILE/Dynabeads M-280 Streptavidin.pdf.
Bio Analyzer User Manual (Agilent): hypertext transfer protocol://world wide web.chem.agilent.com/temp/rad31B29/00033620.pdf
BioAnalyzer DNA and RNA LabChip Usage (Agilent): hypertext transfer protocol://world wide web.agilent.com/chem/labonachip
BioAnalyzer RNA 6000 Ladder (Ambion): hypertext transfer protocol://world wide web.ambion.com/techlib/spec/sp_7152.pdf

Throughout this specification, various patents, published patent applications and scientific references are cited to describe the state and content of the art. Those disclosures, in their entireties, are hereby incorporated into the present specification by reference.

### EMBODIMENTS OF THE DISCLOSURE

1. A method for clonally isolating a library comprising a plurality of single stranded DNA molecules comprising:
   (a) fragmenting large template DNA molecules to generate a plurality of fragmented DNA molecules;
   (b) attaching a first or second universal double stranded adaptor to a first end of each fragmented DNA molecule and a first or second universal adaptor to a second end of each fragmented DNA molecule to form a mixture of adaptor ligated DNA molecules;
   (c) isolating a plurality of single stranded DNA molecules each comprising a first single stranded universal adaptor and a second single stranded universal adaptor to obtain a library; and
   (d) delivering the single stranded DNA molecules into reactors such that a plurality of the reactors include one DNA molecule, thereby clonally isolating the library.
2. A method for generating a library comprising a plurality of single stranded DNA molecules, comprising:
   (a) fragmenting large or whole genomic template DNA molecules to generate a plurality of fragmented DNA molecules;
   (b) ligating a first universal double stranded adaptor or a second universal adaptor to a first end of each fragmented DNA molecule and a first universal adaptor or second universal adaptor to a second end of each fragmented DNA molecule to produce a mixture of adaptor ligated DNA molecules, wherein the first universal adaptor contains a moiety that binds to a solid support;
   (c) attaching to a solid support those DNA molecules comprising a first double stranded universal adaptor;
   (d) removing adaptor ligated DNA molecules which have not attached to a solid support;
   (e) strand separating those adaptor ligated DNA molecules that are attached to a solid support at only one end to release a plurality of single stranded DNA molecules having a first single stranded universal adaptor at one end and a second single stranded adaptor at the other end; and
   (f) isolating a library of the released single stranded DNA molecules of step (e) away from those DNA molecules that remain attached to the solid support.
3. The method according to Embodiment 1 or 2, wherein the first universal double stranded adaptor or the second universal adaptor is attached by ligation.
4. The method according to Embodiment 1, wherein (f) is accomplished by: i) delivering the single stranded DNA molecules onto a location on a reactor array; or ii) delivering the single stranded DNA molecules into droplets in a water-in-oil emulsion.
5. The method according Embodiment 1 or 2, further comprising the step of repairing single stranded nicks in the mixture of adaptor ligated DNA molecules using DNA repair and modifying enzymes.
6. The method according to Embodiment 5, wherein the DNA repair and modifying enzymes are selected from the group consisting of polymerase, ligase, kinase, and combinations thereof.
7. The method according to Embodiment 6, wherein the polymerase is *Bacillus stearothermophilus* polymerase I, the ligase is T4 ligase, and the kinase is T4 polynucleotide kinase.
8. The method according to Embodiment 1 or 2, wherein the template DNA is selected from the group consisting of genomic DNA, cDNA, plasmid DNA, cosmid DNA, artificial chromosome DNA, synthetic DNA, phasemid DNA, and phagemid DNA.
9. The method according to Embodiment 1 or 2, wherein the template DNA comprises reverse transcripts.
10. The method according to Embodiment 1 or 2, wherein the fragmenting is performed by a means selected from the group consisting of enzymatic, chemical, and mechanical means.
11. The method according to Embodiment 10, wherein the enzymatic means is DNase I.
12. The method according to Embodiment 10, wherein the mechanical means is nebulization.
13. The method according to Embodiment 11, wherein the DNase I digestion is performed at a temperature of 10-37°C for 1-2 minutes.
14. The method according to Embodiment 10, wherein the enzymatic means is a restriction endonuclease.
15. The method according to Embodiment 10, wherein the mechanical means is selected from the group consisting of a French Press, a sonicator, a HydroShear, and a nebulizer.
16. The method according to Embodiment 1 or 2, wherein the fragmented DNA molecules are 50 bp to 700 bp in length.
17. The method according to Embodiment 1 or 2, wherein the compatible ends are blunt ends.
18. The method according to Embodiment 17 wherein the blunt ends are created with an enzyme selected from the group consisting of *Pfu* polymerase, T4 DNA polymerase and Klenow fragments.
19. The method according to Embodiment 1 or 2, wherein the compatible ends include an A or T overhang.
20. The method according to Embodiment 1 or 2, wherein the first or second double stranded universal adaptor comprises phosphorothioate linkages.
21. The method according to Embodiment 1 or 2, wherein a biotin moiety is attached to the first or second double stranded universal adaptor.
22. The method according to Embodiment 1 or 2, wherein the first or second double stranded universal adaptor is ligated with T4 DNA ligase.
23. The method according to Embodiment 1 or 2, wherein either the first double stranded universal adaptors or the second double stranded universal adaptors or both double stranded universal adaptors comprise a discriminating key sequence.
24. The method according to Embodiment 23, wherein the discriminating key sequence is 3-12 nucleotides in length.
25. The method according to Embodiment 23, wherein the discriminating key sequence comprises at least one nucleotide selected from the group consisting of A, G, C, U, and T.
26. The method according to Embodiment 1 or 2, wherein first and second double stranded universal adaptor comprise a PCR priming sequence and a sequencing primer sequence.
27. The method according to Embodiment 26, wherein the PCR priming sequence is 10-20 base pairs in length.
28. The method according to Embodiment 26 wherein the sequencing primer sequence is 10-20 base pairs in length.
29. The method according to Embodiment 26, wherein the PCR priming sequence and the sequencing primer sequence overlap.
30. The method according to Embodiment 1 or 2, wherein the mixture of adaptor ligated DNA molecules is separated by a method selected from the group consisting of gel electrophoresis, filtration, size exclusion chromatography, and sucrose sedimentation.
31. The method according to Embodiment 1 or 2, wherein the plurality of single stranded DNA molecules is attached to a DNA capture bead.
32. The method according to Embodiment 31, wherein the DNA capture bead comprises a component of a binding pair.
33. The method according to Embodiment 31 wherein the binding pair is selected from the group consisting of avidin/biotin, ligand/receptor, antigen/antibody and complementary nucleotides.
34. The method according to Embodiment 31, wherein the DNA capture bead is a paramagnetic bead.
35. The method according to Embodiment 1 or 2, wherein the plurality of single stranded DNA molecules is obtained by a treatment selected from the group consisting of low salt treatment, high pH treatment, and chemical denaturation treatment.
36. A method for generating a single stranded DNA library attached to solid supports comprising:
   (a) generating a plurality of single stranded DNA templates;
   (b) attaching each of the plurality of ssDNA templates to a solid support; and
   (c) isolating the solid supports on which the single stranded DNA templates are attached.
37. A method for generating a single stranded DNA library attached to solid supports comprising:
   (a) fragmenting large template DNA molecules to generate a plurality of fragmented DNA molecules;
   (b) attaching a first or second universal double stranded adaptor to a first end of each fragmented DNA molecule and a first or second universal adaptor to a second end of each fragmented DNA molecule to make a mixture of adaptor ligated DNA molecules;
   (c) isolating those single stranded DNA molecules which comprise a first single stranded universal adaptor and a second single stranded universal adaptor; and
   (d) attaching the isolated single stranded molecules from (c) to a solid support.
38. The method according to Embodiment 37, wherein the solid support is a DNA capture bead.
39. The method according to Embodiment 37, wherein the DNA is selected from the group consisting of genomic DNA, cDNA, plasmid DNA, cosmid DNA, artificial chromosome DNA, synthetic DNA, phasemid DNA, and phagemid DNA.
40. The method according to Embodiment 37, wherein the DNA is attached to the solid support via a binding pair.
41. The method according to Embodiment 40, wherein the binding pair is selected from the group consisting of avidin/biotin, ligand/receptor, antigen/antibody and complementary nucleotides.
42. A library of mobile solid supports made by the method of claim 37.
43. A nucleic acid molecule comprising a first adaptor, a fragment of template DNA, and a second adaptor, wherein the first adaptor and second adaptor each comprise a sequencing primer, a PCR primer, and a discriminating key sequence, and wherein the first adaptor and second adaptor, when dissociated, do not cross-hybridize to each other under stringent hybridization conditions.
44. The nucleic acid molecule of Embodiment 43, wherein the PCR primer is 10-20 base pairs in length.
45. The nucleic acid molecule of Embodiment 43, wherein the sequencing primer is 10-20 base pairs in length.
46. The nucleic acid molecule of Embodiment 43, wherein the discriminating key sequence is 3 to 12 base pairs in length.
47. The nucleic acid molecule of Embodiment 43, wherein the template DNA is selected from the group consisting of genomic DNA, cDNA plasmid DNA, cosmid DNA, artificial chromosome DNA, synthetic DNA, phasemid DNA, and phagemid DNA.
48. The nucleic acid molecule of Embodiment 43, wherein the nucleic acid molecule, when dissociated, has minimal cross-hybridization to dissociated template DNA.
49. A method for preparing single stranded DNA molecules, comprising:
   (a) fragmenting large or whole genomic template DNA molecules to generate a plurality of fragmented DNA molecules;
   (b) ligating a first universal double stranded adaptor or a second universal adaptor to a first end of each fragmented DNA molecule and a first universal adaptor or second universal adaptor to a second end of each fragmented DNA molecule to produce a mixture of adaptor ligated DNA molecules;
   (c) attaching adaptor ligated DNA molecules comprising a first double stranded universal adaptor and a second double stranded adaptor to a solid support via one strand of the first double stranded universal adaptor;
   (d) washing away adaptor ligated DNA molecules which have not attached to a solid support;
   (e) strand separating those adaptor ligated DNA molecules that are attached to a solid support at only one end to release a plurality of single stranded DNA molecules comprising a first single stranded universal adaptor at one end and a second single stranded adaptor at the other end; and; and
   (f) isolating the single stranded DNA molecules.
50. A method for delivering nucleic acid templates to a plurality of reaction centers comprising the steps of:
   (a) providing a population of nucleic acid templates;
   (b) isolating each nucleic acid template from said population to a sequestering agent to form a population of sequestered nucleic acid templates;
   (c) delivering said population of sequestered nucleic acid templates to said plurality of reaction centers wherein each reaction center receives one sequestered nucleic acid.
51. The method of Embodiment 50, wherein said isolating step comprises attaching said nucleic acid templates to a bead.
52. The method of Embodiment 50, wherein said isolating step comprises encapsulating said nucleic acid template in an emulsion of a water-in-oil emulsion.
53. The method of Embodiment 52, wherein said nucleic acid template is encapsulated with a bead and wherein the bead can bind said nucleic acid.
54. The method of Embodiment 50, wherein said delivering step comprises delivering said sequestered nucleic acid to a plurality of reaction centers, wherein each reaction center is a well on a picotiter plate.
55. The method of Embodiment 1, 2 or 49 further comprising the step of attaching the isolated single stranded molecules each individually to a solid support.

## Claims

1. A method for delivering nucleic acid templates to a plurality of reaction centers comprising the steps of:
(a) providing a population of nucleic acid templates;
(b) isolating each nucleic acid template from said population to a sequestering agent to form a population of sequestered nucleic acid templates;
(c) delivering said population of sequestered nucleic acid templates to said plurality of reaction centers wherein each reaction center receives one sequestered nucleic acid.

2. The method of claim 1, wherein said isolating step comprises attaching said nucleic acid templates to a bead.

3. The method of claim 1, wherein said isolating step comprises encapsulating said nucleic acid template in an emulsion of a water-in-oil emulsion.

4. The method of claim 3, wherein said nucleic acid template is encapsulated with a bead and wherein the bead can bind said nucleic acid.

5. The method of claim 1, wherein said delivering step comprises delivering said sequestered nucleic acid to a plurality of reaction centers, wherein each reaction center is a well on a picotiter plate.

6. A method for clonally isolating a library comprising a plurality of single stranded DNA molecules comprising:
(a) fragmenting large template DNA molecules to generate a plurality of fragmented DNA molecules;
(b) attaching a first or second universal double stranded adaptor to a first end of each fragmented DNA molecule and a first or second universal adaptor to a second end of each fragmented DNA molecule to form a mixture of adaptor ligated DNA molecules;
(c) isolating a plurality of single stranded DNA molecules each comprising a first single stranded universal adaptor and a second single stranded universal adaptor to obtain a library; and
(d) delivering the single stranded DNA molecules into reactors such that a plurality of the reactors include one DNA molecule, thereby clonally isolating the library.

7. A method for generating a library comprising a plurality of single stranded DNA molecules, comprising:
(a) fragmenting large or whole genomic template DNA molecules to generate a plurality of fragmented DNA molecules;
(b) ligating a first universal double stranded adaptor or a second universal adaptor to a first end of each fragmented DNA molecule and a first universal adaptor or second universal adaptor to a second end of each fragmented DNA molecule to produce a mixture of adaptor ligated DNA molecules, wherein the first universal adaptor contains a moiety that binds to a solid support;
(c) attaching to a solid support those DNA molecules comprising a first double stranded universal adaptor;
(d) removing adaptor ligated DNA molecules which have not attached to a solid support;
(e) strand separating those adaptor ligated DNA molecules that are attached to a solid support at only one end to release a plurality of single stranded DNA molecules having a first single stranded universal adaptor at one end and a second single stranded adaptor at the other end; and
(f) isolating a library of the released single stranded DNA molecules of step (e) away from those DNA molecules that remain attached to the solid support.

8. A method for generating a single stranded DNA library attached to solid supports comprising: (a) generating a plurality of single stranded DNA templates; (b) attaching each of the plurality of ssDNA templates to a solid support; and (c) isolating the solid supports on which the single stranded DNA templates are attached.

9. A method for generating a single stranded DNA library attached to solid supports comprising:
(a) fragmenting large template DNA molecules to generate a plurality of fragmented DNA molecules;
(b) attaching a first or second universal double stranded adaptor to a first end of each fragmented DNA molecule and a first or second universal adaptor to a second end of each fragmented DNA molecule to make a mixture of adaptor ligated DNA molecules;
(c) isolating those single stranded DNA molecules which comprise a first single stranded universal adaptor and a second single stranded universal adaptor; and
(d) attaching the isolated single stranded molecules from (c) to a solid support.

10. A library of mobile solid supports made by the method of claim 9.

11. A nucleic acid molecule comprising a first adaptor, a fragment of template DNA, and a second adaptor, wherein the first adaptor and second adaptor each comprise a sequencing primer, a PCR primer, and a discriminating key sequence, and wherein the first adaptor and second adaptor, when dissociated, do not cross-hybridize to each other under stringent hybridization conditions.

12. A method for preparing single stranded DNA molecules, comprising:
(a) fragmenting large or whole genomic template DNA molecules to generate a plurality of fragmented DNA molecules;
(b) ligating a first universal double stranded adaptor or a second universal adaptor to a first end of each fragmented DNA molecule and a first universal adaptor or second universal adaptor to a second end of each fragmented DNA molecule to produce a mixture of adaptor ligated DNA molecules;
(c) attaching adaptor ligated DNA molecules comprising a first double stranded universal adaptor and a second double stranded adaptor to a solid support via one strand of the first double stranded universal adaptor;
(d) washing away adaptor ligated DNA molecules which have not attached to a solid support;
(e) strand separating those adaptor ligated DNA molecules that are attached to a solid support at only one end to release a plurality of single stranded DNA molecules comprising a first single stranded universal adaptor at one end and a second single stranded adaptor at the other end; and; and
(f) isolating the single stranded DNA molecules.
